# EUROPEAN PATENT APPLICATION

(11) **EP 4 205 694 A1**
(43) Date of publication of application: **05.07.2023**
(21) Application number: 21860017.9
(22) Date of filing: 27.07.2021
(51) Int. Cl.: A61B 90/50, A61B 34/30, A61B 17/00

(54) **SURGICAL INSTRUMENT AND SURGICAL INSTRUMENT PLATFORM**

(30) Priority: 26.08.2020 CN 202010872347
(71) Applicant: Shanghai Microport Medbot (Group) Co., Ltd., Shanghai 201203 (CN)
(72) Inventor: LI, Tao, Shanghai 201203 (CN); HE, Chao, Shanghai 201203 (CN); ZHOU, Jinlong, Shanghai 201203 (CN)
(74) Representative: Patentship Patentanwaltsgesellschaft mbH
(86) International application number: PCT/CN2021/108599
(87) International publication number: WO 2022/042177

(57) **Abstract**

The present invention relates to a surgical instrument platform comprising a base, an adjustment assembly and an instrument assembly. The adjustment assembly is disposed on the base and comprises a mechanical arm. The instrument assembly comprises a surgical instrument and an instrument platform. The surgical instrument is detachably disposed on the instrument platform. The instrument platform is coupled to an end of the mechanical arm. The surgical instrument comprises, connected sequentially, a manipulation end, a connection assembly and an end effector, as well as, a sensor member, a drive member, a transmission member and a control member. The connection assembly comprises first and second connecting structures. A proximal end of the first connecting structure is coupled to the manipulation end and a distal end of the first connecting structure is coupled to the instrument platform. A distal end of the second connecting structure is coupled to the end effector and a proximal end of the second connecting structure is coupled to the instrument platform. The sensor member detects movement of the first connecting structure, and the control member controls the drive member based on the detected movement of the first connecting structure to drive movement of the second connecting structure through the transmission member. The present invention is advantageous in enabling more precise, more reliable, more labor-saving and more convenient surgical operation.

## Description

### TECHNICAL FIELD

The present invention relates to the field of medical instruments and, in particular, to a surgical instrument platform.

### BACKGROUND

As minimally invasive surgical robot systems are being increasingly used in clinical practice, patients become more and more demanding on minimally invasive surgery, especially in terms of the number and location of incisions. Single-incision surgery allows fewer incisions to be made and can additionally reduce surgical trauma, thus decreasing the required anaesthetic or analgesic substance, patients' postoperative pain and the risk of incision infection and complications. Moreover, scars resulting from single-incision surgery are less aesthetically obtrusive. However, this surgery requires nearly parallel introduction of surgical instruments into the abdominal cavity, leading to difficulties in ensuring a triangular working space and in performing pulling and exposing operations. Moreover, ordinary surgical instruments that are simultaneously operating may cross or rub each other. This not only affects their normal use but may also cause secondary damage to the patient in some cases.

Chinese patent application CN105266856A discloses a glove-like single-incision laparoscopic puncture device adopting a soft glove-like structure. Although this device allows a larger surgeon-side working space, it fails to overcome the problem of mutually interfering surgical instruments in the abdominal cavity. Chinese patent application CN104352264A discloses a laparoscopic surgical instrument with multiple degrees of freedom, which includes a handle provided with a rotary wheel. A push pod passes proximally through a center of the rotary wheel so that a bendable unit disposed at a leading end of the instrument can be controlled to bend in any desired direction by appropriately rotating the rotary wheel. However, this instrument requires an operator to manipulate the push pod separately, making it difficult for the operator to adjust the instrument at any time desired. Moreover, movement of the push pod causes opposite movement of an end effector, adding operating difficulties. Further, further improvement would be desirable for this instrument in terms of comfort and convenience in surgical operation.

### SUMMARY OF THE INVENTION

In order to overcome one or more of the above problems, the present invention provides a surgical instrument platform, which enables more labor-saving and convenient surgical operation, more precise motion control, interference-free operation of two or more surgical instruments/endoscopes both or all inserted in a human body and less surgical difficulties.

To this end, a surgical instrument platform provided in the present invention comprises: a base; an adjustment assembly disposed on the base and comprising a mechanical arm; and an instrument assembly comprising a surgical instrument and an instrument platform, the surgical instrument detachably disposed on the instrument platform, the instrument platform coupled to an end of the mechanical arm so as to enable the mechanical arm to adjust a position and an orientation of the instrument assembly,
wherein: the surgical instrument comprises a manipulation end, a connection assembly and an end effector, which are connected sequentially from a proximal end to a distal end of the surgical instrument; the connection assembly comprises a proximal first connecting structure and a distal second connecting structure; a proximal end of the first connecting structure is coupled to the manipulation end, and a distal end of the first connecting structure is coupled to the instrument platform; a distal end of the second connecting structure is coupled to the end effector, and a proximal end of the second connecting structure is coupled to the instrument platform; and wherein: the surgical instrument further comprises a sensor member, a drive member, a transmission member and a control member; the sensor member is configured to detect a movement of the first connecting structure and to send a detected movement information of the first connecting structure to the control member; the control member is configured to control the drive member to output a power based on the detected movement information of the first connecting structure; and the drive member is configured to drive a movement of the second connecting structure through the transmission member.

Optionally, the first connecting structure may comprise a first flexible structure and the second connecting structure may comprise a second flexible structure, each of the first and second flexible structures having at least one rotational degree of freedom, wherein: the sensor member is further configured to detect a rotation of the first flexible structure and to send a detected rotation information to the control member; the control member is further configured to control the drive member to output a power based on the detected rotation information of the first flexible structure; and the drive member is further configured to drive, through the transmission member, the second flexible structure to follow the rotation of the first flexible structure to rotate in an opposite direction.

Optionally, the connection assembly may further comprise a third flexible structure, wherein the third flexible structure is disposed between the second connecting structure and the end effector and has at least one rotational degree of freedom for driving the end effector to rotate, and wherein the manipulation end comprises a manipulator structure, a wrist structure and a grip structure that are sequentially connected, wherein the manipulator structure is disposed on the wrist structure and is configured to drive the wrist structure to move, wherein the grip structure is coupled to the proximal end of the first connecting structure, the wrist structure having at least one rotational degree of freedom, wherein: the sensor member is configured to detect a rotation of the wrist structure and to send a detected rotation information to the control member; the control member is configured to control the drive member to output a power based on the detected rotation information of the wrist structure; and the drive member is configured to drive, through the transmission member, the third flexible structure to follow the rotation of the wrist structure to rotate in an opposite direction.

Optionally, the first connecting structure may comprise a first outer tube and a first inner tube and the second connecting structure may comprise a second outer tube and a second inner tube, the first outer tube nesting on the first inner tube, the second outer tube nesting on the second inner tube, wherein: a proximal end of the first inner tube is coupled to the manipulation end; a distal end of the first inner tube is a free end and is configured to be inserted into the first outer tube; a proximal end of the first outer tube is a free end and is positioned outside of the instrument platform; and a distal end of the first outer tube is coupled to the instrument platform, wherein: a distal end of the second inner tube is coupled to the end effector; a proximal end of the second inner tube is a free end and is configured to be inserted into the second outer tube; a proximal end of the second outer tube is coupled to the instrument platform; and a distal end of the second outer tube is a free end and is positioned outside of the instrument platform, wherein: the first outer tube is provided therein with the first flexible structure; and the second outer tube is provided therein with the second flexible structure.

Optionally, the distal end of the second inner tube may be coupled to the end effector by a third flexible structure having at least one rotational degree of freedom; and/or each of the distal end of the first inner tube and the proximal end of the second inner tube extends into the instrument platform.

Optionally, the first flexible structure may have two rotational degrees of freedom respectively about a first axis and a second axis, and the second flexible structure may have two rotational degrees of freedom respectively about a third axis and a fourth axis, wherein: the third axis is parallel to the first axis; the fourth axis is parallel to the second axis; and the first axis is perpendicular to the second axis, wherein when the first flexible structure is being driven to rotate about the first axis, the sensor member is configured to detect the rotation of the first flexible structure about the first axis and to send a first detected rotation information to the control member; the control member is configured to control the drive member to output a power based on the first detected rotation information; and the drive member is configured to drive, through the transmission member, the second flexible structure to rotate about the third axis in an opposite direction to the rotation of the first flexible structure, and wherein: when the first flexible structure is being driven to rotate about the second axis, the sensor member is configured to detect the rotation of the first flexible structure about the second axis and send a second detected rotation information to the control member; the control member is configured to control the drive member to output power based on the second detected rotation information; and the drive member is configured to drive, through the transmission member, the second flexible structure to rotate about the fourth axis in an opposite direction to the rotation of the first flexible structure.

Optionally, the first inner tube may be able to translate relative to the first outer tube and the second inner tube may be able to translate relative to the second outer tube, wherein: the sensor member is further configured to detect a translation of the first inner tube and/or the manipulation end and to send a detected translation information of the first inner tube and/or the manipulation end to the control member; the control member is configured to control the drive member to output a power based on a detected translation information of the first inner tube and/or the manipulation end; and the drive member is configured to drive the second inner tube to translate through the transmission member.

Optionally, the first inner tube may comprise a first proximal segment, a first intermediate segment and a first distal segment, which are connected sequentially from a proximal end to a distal end of the first inner tube, wherein the first proximal segment is coupled to the manipulation end, wherein each of the first proximal segment and the first distal segment is implemented as a rigid segment, the first intermediate segment implemented as a flexible segment, and wherein the first outer tube comprises a first proximal section, the first flexible structure and a first distal section, which are connected sequentially from a proximal end to a distal end of the first outer tube, the first distal section coupled to the instrument platform, wherein an aggregate axial length of the first proximal segment and the first intermediate segment is greater than an aggregate axial length of the first proximal section and the first flexible structure, and wherein the first distal segment is accommodated in the first distal section and extends into the instrument platform.

Optionally, a proximal portion of the first proximal segment may comprise a first inner sub-tube and a second inner sub-tube, wherein the first inner sub-tube has an outer diameter that is not greater than an inner diameter of the first proximal section, wherein the second inner sub-tube has an outer diameter that is greater than the inner diameter of the first proximal section, wherein an aggregate axial length of the first inner sub-tube and the first intermediate segment is greater than the aggregate axial length of the first proximal section and the first flexible structure.

Optionally, the second inner tube may comprise a second proximal segment, a second intermediate segment and a second distal segment, which are connected sequentially from a proximal end to a distal end of the second inner tube, wherein each of the second proximal segment and the second distal segment is implemented as a rigid segment, the second intermediate segment implemented as a flexible segment, wherein the second distal segment is coupled to the end effector, and wherein the second proximal segment extends into the instrument platform and is coupled to the drive member, and wherein the second outer tube comprises a second proximal section, the second flexible structure and a second distal section, which are connected sequentially from a proximal end to a distal end of the second outer tube, wherein the second proximal section is coupled to the instrument platform, wherein an aggregate axial length of the second intermediate segment and the second distal segment is greater than an aggregate axial length of the second flexible structure and the second distal section.

Optionally, a distal portion of the second distal segment may consist of a third inner sub-tube and a fourth inner sub-tube, wherein the third inner sub-tube has an outer diameter that is not greater than an inner diameter of the second distal section, wherein the fourth inner sub-tube has an outer diameter that is greater than the inner diameter of the second distal section, and wherein an aggregate axial length of the third inner sub-tube and the second intermediate segment is greater than the aggregate axial length of the second distal section and the second flexible structur.

Optionally, the wrist structure may have two rotational degrees of freedom respectively about a fifth axis and a sixth axis, and the third flexible structure may have two rotational degrees of freedom respectively about a seventh axis and an eighth axis, wherein: the fifth axis is parallel to the seventh axis; the sixth axis is parallel to the eighth axis; and the fifth axis is perpendicular to the sixth axis, wherein when the wrist structure is being driven by the manipulator structure to rotate about the fifth axis, the sensor member is configured to detect the rotation of the wrist structure about the fifth axis and to send a third detected rotation information to the control member, wherein the control member is configured to control the drive member to output a power based on the third detected rotation information, and wherein the drive member is configured to drive, through the transmission member, the third flexible structure to rotate about the seventh axis in a same direction as the rotation of the wrist structure, and wherein when the wrist structure is being driven by the manipulator structure to rotate about the sixth axis, the sensor member is configured to detect the rotation of the wrist structure about the sixth axis and to send a fourth detected rotation information to the control member, wherein the control member is configured to control the drive member to output a power based on the fourth detected rotation information, and wherein the drive member is configured to drive, through the transmission member, the third flexible structure to rotate about the eighth axis in a same direction as the rotation of the wrist structure.

Optionally, the manipulator structure may have a roll degree of freedom about a ninth axis, wherein the end effector has a roll degree of freedom about a tenth axis, wherein the manipulator structure is rotatably disposed on the wrist structure and wherein the end effector is coupled to the third flexible structure, wherein when the manipulator structure is rolling about the ninth axis, the sensor member is configured to detect the rolling of the manipulator structure and to send a detected rolling information of the manipulator structure to the control member, wherein the control member is configured to control the drive member to output a power based on the detected rolling information of the manipulator structure, and wherein the drive member is configured to drive, through the transmission member, the end effector to roll in a same direction as the rolling of the manipulator structure.

Optionally, the end effector may comprise at least one tool arm and the manipulation end may further comprise an opening and closing control structure disposed on the manipulator structure and being able to make opening and closing movement, wherein when the opening and closing control structure is making the opening and closing movement, the sensor member is configured to detect the opening and closing movement of the opening and closing control structure and to send the detected opening and closing movement information to the control member, wherein the control member is configured to control the drive member to output a power based on the detected opening and closing movement information, wherein the drive member is configured to drive, through the transmission member, the tool arm of the end effector to make an opening and closing movement, which occurs in a same manner as the opening and closing movement of the opening and closing control structure..

Optionally, the sensor member may comprise a number of sensors for detecting movement of each of the manipulation end and the first flexible structure.

Optionally, the sensor member may comprise a first sensor, a second sensor, a third sensor, a fourth sensor, a fifth sensor, a sixth sensor and a seventh sensor, wherein: the first sensor is configured to detect a pitch movement of the wrist structure; the second sensor is configured to detect a yaw movement of the wrist structure; the third sensor is configured to detect the opening and closing movement of the manipulation end; the fourth sensor is configured to detect a roll movement of the manipulator structure; the fifth sensor is configured to detect a pitch movement of the first flexible structure; the sixth sensor is configured to detect a yaw movement of the first flexible structure; and the seventh sensor is configured to detect a translational movement of the manipulation end.

Optionally, the transmission member may comprise a first transmission part and a second transmission part, wherein: the first transmission part is configured to convert a movement of the first connecting structure into a movement detectable by the sensor member; and the second transmission part is coupled to the drive member to drive the second connecting structure to move.

Optionally, the first transmission part may comprise a first cable transmission assembly and a second cable transmission assembly, wherein the first cable transmission assembly comprises a first transmission cable and a first cable pulley, wherein the first cable transmission assembly is configured to use the first transmission cable to convert a pitch movement of the first flexible structure about a first axis into a rotational movement of the first cable pulley, wherein the second cable transmission assembly comprises a second transmission cable and a second cable pulley, wherein the second cable transmission assembly is configured to use the second transmission cable to convert a yaw movement of the first flexible structure about a second axis into a rotational movement of the second cable pulley, wherein: a proximal end of the first transmission cable is coupled to the first flexible structure; a distal end of the first transmission cable is wound through the first cable pulley and is then coupled to the first flexible structure; a proximal end of the second transmission cable is coupled to the first flexible structure; and a distal end of the second transmission cable is wound through the second cable pulley and is then coupled to the first flexible structure, wherein the sensor member is configured to detect the rotational movement of the first cable pulley and the rotational movement of the second cable pulley and to send a detected rotation information of the first cable pulley and a detected rotation information of the second cable pulley to the control member, wherein the control member is configured to determine a rotational condition of the first flexible structure about a first axis based on the detected rotation information of the first cable pulley and to determine a rotational condition of the first flexible structure about a second axis based on the detected rotation information of the second cable pulley.

Optionally, the first transmission part may be also configured to convert movement of the wrist structure into a movement detectable by the sensor member, wherein the first transmission part comprises a third cable transmission assembly and a fourth cable transmission assembly, wherein the third cable transmission assembly comprises a third transmission cable and a third cable pulley, wherein the third cable transmission assembly is configured to use the third transmission cable to convert a pitch movement of the wrist structure about a fifth axis into a rotational movement of the third cable pulley, wherein a proximal end of the third transmission cable is coupled to the wrist structure, and wherein a distal end of the third transmission cable is wound through the third cable pulley and is then coupled to the wrist structure, wherein the fourth cable transmission assembly comprises a fourth transmission cable and a fourth cable pulley, wherein the fourth cable transmission assembly is configured to use the fourth transmission cable to convert a yaw movement of the wrist structure about a sixth axis into a rotational movement of the fourth cable pulley, wherein a proximal end of the fourth transmission cable is coupled to the wrist structure, and wherein a distal end of the fourth transmission cable is wound through the fourth cable pulley and is then coupled to the wrist structure, wherein the sensor member is configured to detect the rotational movement of the third cable pulley and the rotational movement of the fourth cable pulley, and to send a detected rotation information of the third cable pulley and a detected rotation information of the fourth cable pulley to the control member, wherein the control member is configured to determine a rotational condition of the wrist structure about the fifth axis based on the detected rotation information of the third cable pulley and to determine a rotational condition of the wrist structure about the sixth axis based on the detected rotation information of the fourth cable pulley.

Optionally, the drive member may comprise a first motor and a second motor and the second transmission part may comprise a fifth cable transmission assembly and a sixth cable transmission assembly, wherein the fifth cable transmission assembly is configured to drive a pitch movement of the second flexible structure, and wherein the sixth cable transmission assembly is configured to drive a yaw movement of the second flexible structure, wherein the fifth cable transmission assembly comprises at least two fifth transmission cables, wherein a proximal end of each of the fifth transmission cables is coupled to the first motor and a distal end of each of the fifth transmission cables is coupled to the second flexible structure, wherein the sixth cable transmission assembly comprises at least two sixth transmission cables, wherein a proximal end of each of the sixth transmission cables is coupled to the second motor and a distal end of each of the sixth transmission cables is coupled to the second flexible structures.

Optionally, the drive member may comprise a third motor and a fourth motor and the second transmission part may comprise a seventh cable transmission assembly and an eighth cable transmission assembly, the seventh cable transmission assembly configured to drive pitch movement of the third flexible structure, the eighth cable transmission assembly configured to drive yaw movement of the third flexible structure, wherein the seventh cable transmission assembly comprises at least two seventh transmission cables, wherein a proximal end of each of the seventh transmission cables is coupled to the third motor, and wherein a distal end of each of the seventh transmission cables passes through the second flexible structure and is then coupled to the third flexible structure, wherein the eighth cable transmission assembly comprises at least two eighth transmission cables, wherein a proximal end of each of the eighth transmission cables is coupled to the fourth motor, and wherein a distal end of each of the eighth transmission cables passes through the second flexible structure and is then coupled to the third flexible structure.

Optionally, the first transmission part may comprise a first rack and pinion transmission mechanism comprising a first rack and a first pinion in meshing engagement with the first rack, wherein the first pinion is coupled to the drive member, wherein the first rack is coupled to the first connecting structure, and wherein the first rack and pinion transmission mechanism is configured to convert a translation of the first connecting structure into a rotational movement of the first pinion, wherein the sensor member is configured to detect the rotational movement of the first pinion and to send a detected rotation information of the first pinion to the control member, wherein the control member is configured to control the drive member to output a power based on the detected rotation information of the first pinion, and wherein the drive member is configured to drive the second connecting structure to move through the second transmission part.

Optionally, the first transmission part may be further configured to convert roll movement of the manipulator structure into a movement detectable by the sensor member, and wherein the first transmission part comprises a first shaft assembly comprising a first flexible shaft and a first rotary wheel, wherein a proximal end of the first flexible shaft is coupled to the manipulator structure, a distal end of the first flexible shaft coupled to the first rotary wheel, wherein the sensor member is configured to detect a rotation of the first rotary wheel and to send a detected rotation information of the first rotary wheel to the control member, wherein the control member is configured to control the drive member to output a power based on the detected rotation information of the first rotary wheel, and wherein the drive member is configured to drive the end effector to roll through the second transmission part.

Optionally, the second transmission part may comprise a second rack and pinion transmission mechanism comprising a second rack and a second pinion in meshing engagement with the second rack, wherein the second pinion is coupled to the drive member, and wherein the second rack is coupled to the second connecting structure, wherein the sensor member is configured to detect a rotation of the second pinion and to send a detected rotation information of the second pinion to the control member, wherein the control member is configured to control the drive member to output a power based on the detected rotation information of the second pinion, and wherein the drive member is configured to drive the second connecting structure to move through the second rack.

Optionally, the second transmission part may comprise a second shaft assembly comprising a second flexible shaft, wherein a proximal end of the second flexible shaft is coupled to the drive member and a distal end of the second flexible shaft is coupled to the end effector, wherein the sensor member is configured to detect a roll movement of the manipulator structure and to send a detected roll information of the manipulator structure to the control member, wherein the control member is configured to control the drive member to output a power based on the detected roll information of the manipulator structure, and wherein the drive member is configured to transmit the roll movement to the end effector through the second flexible shaft, thereby driving the end effector to roll.

Optionally, the second transmission part may comprise a first adapter, a flexible transmission mechanism for opening and closing movement and a second adapter that are connected sequentially, wherein the first adapter is coupled to the drive member and is configured to convert a rotational movement of the drive member into an axial movement of the flexible transmission mechanism, wherein the second adapter is coupled to a tool arm of the end effector and is configured to convert the axial movement of the flexible transmission mechanism into an opening and closing movement of the tool arm, and wherein the flexible transmission mechanism, the first adapter and the second adapter are configured to cause the tool arm of the end effector to move in a same manner as the opening and closing control structure, wherein the sensor member is configured to detect an opening and closing movement of the opening and closing control structure and to send a detected opening and closing information of the opening and closing control structure to the control member, wherein the control member is configured to control the drive member to output a power based on the detected opening and closing information of the opening and closing control structure, and wherein the drive member is configured to drive the opening and closing movement of the end effector through the first adapter, the flexible transmission mechanism and the second adapter.

Optionally, the second cable transmission assembly may further comprise an elastic element for compensating for curvature of the second cable transmission assembly and thereby maintaining the surgical instrument in a deployed configuration.

Optionally, the instrument platform may comprise a housing, on which an interface coupled to the end of the mechanical arm is formed.

Optionally, the mechanical arm may comprise at least six joints to provide at least six degrees of freedom, wherein the mechanical arm includes at least one translational joint and at least five rotational joints, wherein two rotational joints and one translational joint work together to adjust the position of the instrument assembly, and wherein three other rotational joints are configured to adjust the orientation of the instrument assembly.

Optionally, the mechanical arm may comprise seven joints coupled sequentially from a proximal end to a distal end of the mechanical arm, which are a first translational joint, a first rotational joint, a second rotational joint, a third rotational joint, a second translational joint, a fourth rotational joint and a fifth rotational joint, wherein: the first translational joint has an axis of translation that is collinear with an axis of rotation of the first rotational joint; the axis of rotation of the first rotational joint is parallel to an axis of rotation of the second rotational joint; the third rotational joint has an axis of rotation that is collinear with an axis of translation of the second translational joint; and the fourth rotational joint has an axis of rotation that is perpendicular to an axis of rotation of the fifth rotational joint.

Optionally, the axes of rotation of the third, fourth and fifth rotational joints may intersect at a single point.

Optionally, the instrument platform may comprise a housing, on which the surgical instrument is detachably arranged, wherein a sleeve is provided outside of a distal end of the housing, wherein the sleeve extends outwardly from the distal end of the housing, the sleeve configured to be deployed at an incision made in a patient.

Optionally, the housing may be provided therein with a number of instrument channels extending from a proximal end of the housing to a distal end of the sleeve, wherein a proximal portion of the surgical instrument extends into the housing from the proximal end thereof and is accommodated in the corresponding instrument channel in the housing, and wherein a distal portion of the surgical instrument is accommodated in the instrument channel from the distal end thereof and extends through and protrudes out of the sleeve.

Optionally, the instrument platform may further comprises a tool axis, a plane of symmetry and a working plane, wherein the working plane is perpendicular to the plane of symmetry, wherein the working plane crosses axes of at least two of the instrument channels, wherein the instrument channels are arranged in symmetry with respect to the plane of symmetry, and wherein the tool axis is defined as an intersection of the plane of symmetry and the working plane, wherein in an initial configuration, the distal portion of the surgical instrument is linear in shape and is parallel to the tool axis, and wherein the proximal portion of the surgical instrument is curved in shape, and wherein in a deployed configuration, the distal portion of the surgical instrument is C-shaped and is oriented in the working plane, and wherein the proximal portion of the surgical instrument is more curved than in the initial configuration.

A surgical instrument comprises a manipulation end, a connection assembly and an end effector, which are connected sequentially from a proximal end to a distal end of the surgical instrument, wherein: the connection assembly comprises a proximal first connecting structure and a distal second connecting structure; a proximal end of the first connecting structure is coupled to the manipulation end, and a distal end of the first connecting structure is coupled to an instrument platform of a surgical instrument platform; a distal end of the second connecting structure is coupled to the end effector, and a proximal end of the second connecting structure is coupled to the instrument platform, wherein the surgical instrument further comprises a sensor member, a drive member, a transmission member and a control member, wherein: the sensor member is configured to detect a movement of the first connecting structure and to send a detected movement information of the first connecting structure to the control member; the control member is configured to control the drive member to output a power based on the detected movement information of the first connecting structure; and the drive member is configured to drive a movement of the second connecting structure through the transmission member.

The surgical instrument platform of the present invention has at least one of the following advantages:
First, by suspending the instrument assembly on the mechanical arm in the adjustment assembly, more precise, more reliable, more accurate, easier, more convenient and more efficient surgical operation with increased comfort is enabled. Moreover, during surgery, the connection assembly moves under the control of the drive member to drive the end effector to move. This enables more precise movement control of the end effector and hence even more accurate and even more convenient surgical operation with additionally increased comfort.

Second, a linkage is established between the first and second flexible structures in the surgical instrument (for mirrored movement thereof), which enables insertion of two or more surgical instruments into a patient's body for interference-free simultaneous surgical operation. This reduces secondary damage that may be caused to the patient, and the surgical instruments may simultaneously approach the same target tissue to perform the surgical operation within an expanded working space, reducing surgical difficulties.

Third, in the surgical instrument, the third flexible structure allows the manipulation end to move in the same manner as the end effector, thus enabling more intuitive and more convenient surgical operation. Moreover, this can facilitate simultanous approach of two or more surgical instrument to the same target tissue, thus additionally reducing surgical difficulties.

### BRIEF DESCRIPTION OF THE DRAWINGS

Features, aspects and advantages of embodiments of the present invention and related examples will be described with reference to the accompanying drawings, in which:
Fig. 1 is a schematic diagram showing the structure of a surgical instrument platform according to an embodiment of the present invention;
Fig. 2 schematically illustrates degrees of freedom of a surgical instrument platform according to an embodiment of the present invention;
Fig. 3 is a schematic diagram showing the structure of an instrument assembly according to an embodiment of the present invention, which includes two surgical instruments both in an initial configuration;
Fig. 4 is a schematic diagram showing the structure of a gimbal assembly for coupling a second translational arm to an instrument assembly according to an embodiment of the present invention;
Fig. 5 is a schematic diagram showing the structure of an instrument assembly according to an embodiment of the present invention, which includes two surgical instruments both in a deployed configuration;
Fig. 6 is a schematic diagram showing the structure of a surgical instrument according to an embodiment of the present invention, in which an instrument platform is omitted;
Fig. 7a schematically illustrates an outer tube and an inner tube in a connection assembly according to an embodiment of the present invention, which are not assembled together yet;
Fig. 7b schematically illustrates degrees of freedom of an end of a surgical instrument according to an embodiment of the present invention;
Fig. 8a is a schematic diagram showing the structure of an end effector having two pivotal arms according to an embodiment of the present invention;
Fig. 8b is a schematic diagram showing the structure of a manipulator structure provided with an opening and closing control structure according to an embodiment of the present invention;
Fig. 9 is a block diagram showing movement control of a surgical instrument according to an embodiment of the present invention;
Fig. 10 schematically illustrates a flexible element implemented as a snake-arm robotic joint according to an embodiment of the present invention;
Fig. 11a schematically illustrates an end effector in different configuration during its rotation that follows, and occurs in the same direction as, rotation of a manipulation end according to an embodiment of the present invention; and
Fig. 11b schematically illustrates a manipulation end in different configurations during its yaw movement according to an embodiment of the present invention.

### DETAILED DESCRIPTION

The above objects, features and advantages of the present invention will become more apparent and readily appreciated upon reading the following detailed description of some specific embodiments thereof with reference to the accompanying drawings. In the following description, numerous details are set forth in order to provide a thorough understanding of the invention. However, the present invention may be practiced in many other forms than those described herein, and those skilled in the art can make similar improvements without deviating from the spirit of the invention. Accordingly, the present invention is not limited to the specific embodiments disclosed below.

A surgical instrument in the form of a snake-like robotic arm proposed in the present invention will be described below with reference to the accompanying drawings and to some specific embodiments. For ease of understanding, the terms "proximal", "distal" and the like may be used in this application to describe relative orientations, relative positions and directions between components of a medical device or actions thereof, as viewed by a physician operating the device. Without wishing to be limiting, a "proximal end" usually refers to an end closer to the operator, and a "distal end" usually refers to an end farther from the operator, during normal operation of the medical device.

As used herein, the singular forms "a", "an" and "the" are intended to include the plural forms as well, unless the context clearly indicates otherwise. As used herein, the term "or" is generally employed in the sense including "and/or", unless the context clearly dictates otherwise. As used herein, the terms "multiple" and "several" are generally employed in the sense including "two or more than two", unless the context clearly dictates otherwise.

In principle, the present invention seeks to provide a surgical instrument platform including: a base; an adjustment assembly disposed on the base and including a mechanical arm; and an instrument assembly including a surgical instrument and an instrument platform. The surgical instrument is detachably arranged on the instrument platform, and the instrument platform is coupled to an end of the mechanical arm so that the position and orientation of the instrument assembly can be adjusted by the mechanical arm. The surgical instrument includes, sequentially disposed from a proximal end to a distal end thereof, a manipulation end, a connection assembly and an end effector. The connection assembly includes a proximal first connecting structure and a distal second connecting structure. The proximal end of the first connecting structure is coupled to the manipulation end and the distal end of the first connecting structure is coupled to the instrument platform. The distal end of the second connecting structure is coupled to the end effector and the proximal end of the second connecting structure is coupled to the instrument platform. The surgical instrument further includes a sensor member, a drive member, a transmission member and a control member. The sensor member is configured to detect movement of the first connecting structure and send the detected movement information of the first connecting structure to the control member. The control member is configured to control the drive member to output power based on the detected movement information of the first connecting structure. The drive member is configured to drive movement of the second connecting structure by the transmission member.

In a surgical procedure using the above surgical instrument platform, the instrument assembly is suspended on the mechanical arm in the adjustment assembly, enabling more precise, more reliable, more accurate, easier, more convenient and more efficient surgical operation with increased comfort. Moreover, in the surgical procedure, the drive member controls movement of the connection assembly, which in turn causes movement of the end effector. In this way, the end effector can move with increased accuracy, additionally improving accuracy of the surgical operation.

Further, according to the present invention, the first connecting structure includes a first flexible structure, and the second connecting structure includes a second flexible structure. Each of the first and second flexible structures has at least one rotational degree of freedom. The sensor member is configured to detect rotation of the first flexible structure and send the detected rotation information to the control member. The control member is configured to control the drive member to output power based on the detected rotation information of the first flexible structure. The drive member is configured to drive, by the transmission member, the second flexible structure to oppositely rotate with the rotation of the first flexible structure. This design not only facilitates insertion of the surgical instrument in an initial configuration into a patient's body but also allows it to be opened in the patient's body in a controlled manner and easily removed after the surgical procedure is completed. Therefore, the surgical instrument can be more easily mounted, and compared with a pre-curved surgical instrument, expanded working spaces are ensured for both the surgeon and the end effector, facilitating simultaneous surgical operation of two or more instruments on a single target site of target tissue without interference and thereby reducing surgical difficulties.

Furthermore, according to the present invention, the connection assembly further includes a third flexible structure interposed between the second connecting structure and the end effector. The third flexible structure has at least one rotational degree of freedom in order to be able to drive the end effector to rotate therewith. The manipulation end includes a manipulator structure, a wrist structure and a grip structure, which are coupled together sequentially in this order. The proximal end of the first connecting structure is coupled to the grip structure, and the wrist structure has at least one rotational degree of freedom. The sensor member is configured to detect rotation of the wrist structure and send the detected rotation information of the wrist structure to the control member. The control member is configured to control the drive member to output power based on the detected rotation information of the wrist structure. The drive member is configured to drive, by the transmission member, the third flexible structure to rotate with the rotation of the wrist structure in the same direction. As a result, the end effector is caused to rotate together with the manipulation end in the same direction. In this way, two or more surgical instruments are allowed to simultaneously approach the same target tissue, and the manipulation end can move in the same direction as the end effector. This additionally reduces surgical difficulties and lowers surgical risk.

The proposed surgical instrument platform will be described in greater detail below with reference to the accompanying drawings and preferred embodiments.

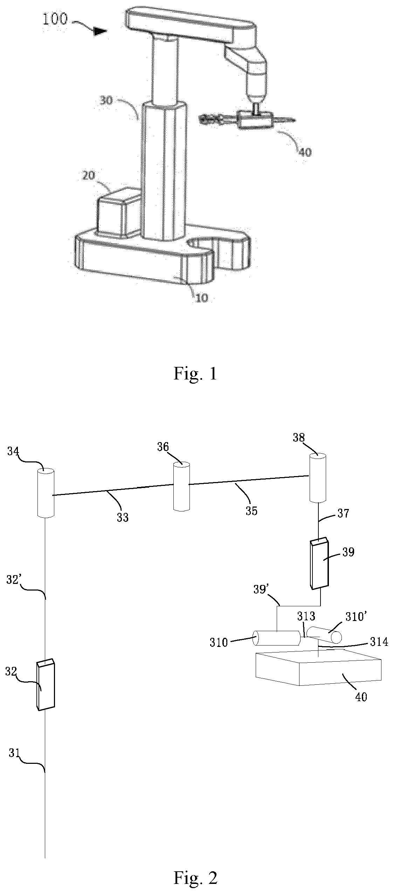

Fig. 1 is a schematic diagram showing the structure of a surgical instrument platform according to an embodiment of the present invention. As shown in Fig. 1, the surgical instrument platform 100 of this embodiment includes a base 10, a balancing assembly 20, an adjustment assembly 30 and an instrument assembly 40. Both the balancing assembly 20 and the adjustment assembly 30 are disposed on the base 10, and the instrument assembly 40 is rotatably coupled (e.g., hinged) to an end of the adjustment assembly 30.

The balancing assembly 20 is configured to balance the instrument assembly 40 and the adjustment assembly 30 so that the surgical instrument platform 100 remains gravitationally balanced during operation. However, the balancing assembly 20 may be alternatively integrated in the adjustment assembly 30. The balancing assembly 20 may be a gravity-balancing mass, or implemented by a motor or constant force spring. In the latter case, the balancing assembly 20 is able to adjust its output, as desired, to accommodate a gravitational change. In this way, the instrument assembly 40 is allowed to have various weights, resulting in greater flexibility and increased applicability of the surgical platform. As gravity balancing is a technique well known to those skilled in the art, the specific implementation of the balancing assembly 20 will not be further described herein.

The adjustment assembly 30 is configured to adjust the position and orientation of the instrument assembly 40. The adjustment assembly 30 includes a mechanical arm with at least six degrees of freedom. An end of the mechanical arm is coupled to the instrument assembly 40 so that the instrument assembly 40 is suspended on the mechanical arm and adjusted by the mechanical arm in position and orientation. This enables more precise, more reliable, more accurate, more labor-saving, more convenient, more efficient, and less strenuous surgical operation, which can be accomplished by a surgeon with increased comfort.

In one embodiment of the present invention, the mechanical arm includes at least six joints, which provides the at least six degrees of freedom. For example, the mechanical arm may include at least one translational joint and at least five rotational joints, in which two rotational joints and one translational joint work together to enable positional adjustability of the instrument assembly 40, and other three rotational joints are coupled to the instrument assembly 40 so as to enable its orientational adjustability. Preferably, the mechanical arm includes at least one other translational joint (e.g., the second translational joint 39 shown in Fig. 2), which serves as a redundant joint for improving adjustment accuracy.

Fig. 2 schematically illustrates degrees of freedom of the mechanical arm according to a first embodiment of the present invention. As shown in Fig. 2, the mechanical arm includes seven joints providing seven degrees of freedom. Specifically, the mechanical arm includes, connected sequentially from a proximal end to a distal end thereof, a first translational joint 32, a first rotational joint 34, a second rotational joint 36, a third rotational joint 38, a second translational joint 39, a fourth rotational joint 310 and a fifth rotational joint 310'. An axis of rotation of the first rotational joint 34 is parallel to an axis of rotation of the second rotational joint 36. An axis of translation of the first translational joint 32 is collinear with the axis of rotation of the first rotational joint 34. An axis of rotation of the third rotational joint 38 is collinear with an axis of translation of the second translational joint 39. An axis of rotation of the fourth rotational joint 310 is perpendicular to an axis of rotation of the fifth rotational joint 310'. Preferably, the axes of rotation of the third rotational joint 38, the fourth rotational joint 310 and the fifth rotational joint 310' intersect at a single point.

Additionally, any adjacent joints are coupled by one arm. For example, the mechanical arm further includes a fixed arm 31, a first translational arm 32', a first rotational arm 33, a second rotational arm 35, a third rotational arm 37, a second translational arm 39', a fourth rotational arm and a fifth rotational arm.

One end of the fixed arm 31 is secured to the base 10, and the other end of the fixed arm 31 is coupled to first translational joint 32. One end of the first translational arm 32' is coupled to the first translational joint 32 so that, by virtue of the first translational joint 32, the first translational arm 32' is able to translate in the direction of the axis of translation of the first translational joint 32 relative to the fixed arm 31. The other end of the first translational arm 32' is coupled to the first rotational joint 34.

One end of the first rotational arm 33 is coupled to the first rotational joint 34 so that the first rotational arm 33 is able to rotate about the axis of rotation of the first rotational joint 34 relative to the first translational arm 32'. The other end of the first rotational arm 33 is coupled to the second rotational joint 36.

One end of the second rotational arm 35 is coupled to the second rotational joint 36 so that the second rotational arm 35 is able to rotate about the axis of rotation of the second rotational joint 36 relative to the first rotational arm 33. The other end of the second rotational arm 35 is coupled to the third rotational joint 38.

One end of the third rotational arm 37 is coupled to the third rotational joint 38 so that the third rotational arm 37 is able to rotate about the axis of rotation of the third rotational joint 38 relative to the second rotational arm 35. The other end of the third rotational arm 37 is coupled to the second translational joint 39.

One end of the second translational arm 39' is coupled to the second translational joint 39 so that, by virtue of the second translational joint 39, the second translational arm 39' is able to translate relative to the third rotational arm 37. The fourth rotational joint 310 is coupled to both the other end of the second translational arm 39' and the fourth rotational arm (which is, for example, an outer gimbal 313). The fifth rotational joint 310' is coupled to both the fourth rotational arm and the fifth rotational arm (which is, for example, an inner gimbal 314). Additionally, the fifth rotational arm is coupled to the instrument assembly 40. In this way, the second translational arm 39' is rotatably coupled to the fourth rotational arm by the fourth rotational joint 310, and the fourth rotational arm is rotatably coupled to the fifth rotational arm by the fifth rotational joint 310'. Thus, the instrument assembly 40 is capable of roll, pitch and yaw motions in the base's coordinate system, which enable orientational adjustability relative to the fixed arm 31.

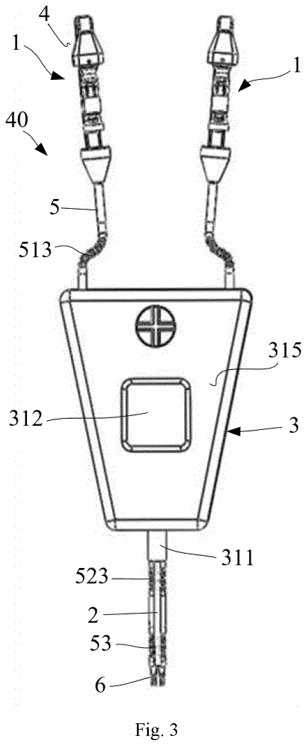

Fig. 3 is a structural schematic of the instrument assembly 40 according to the first embodiment of the present invention. As shown in Fig. 3, in addition to the surgical instrument 1 and the instrument platform 3, the instrument assembly 40 further includes an endoscope 2. Both the surgical instrument 1 and the endoscope 2 are detachably provided on the instrument platform 3. In this embodiment, the instrument platform 3 has an interface 312, which detachably couples the instrument platform 3 to the fifth rotational arm, thereby coupling the instrument assembly 40 to the adjustment assembly 30. Specifically, the fourth rotational joint 310 is coupled to both the second translational arm 39' and the fourth rotational arm, and the fifth rotational joint 310' is coupled to both the fourth rotational arm and the fifth rotational arm, thus allowing the instrument platform 3 to rotate relative to the second translational arm 39' and thereby drive the entire instrument assembly 40 to rotate (e.g., pitch or yaw). Preferably, the fourth rotational joint 310, the fifth rotational joint 310', the fourth rotational arm and the fifth rotational arm are constituent components of a gimbal assembly.

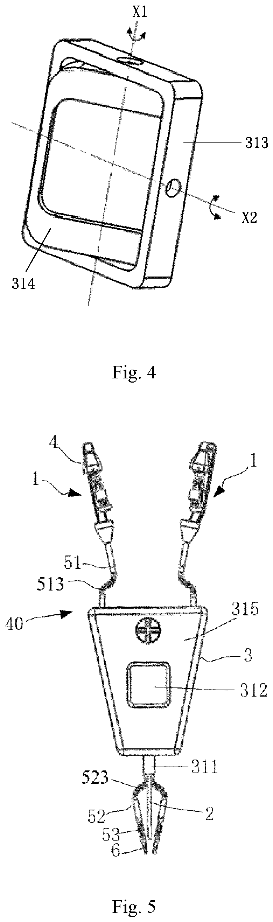

Fig. 4 is a structural schematic of the gimbal assembly according to the first embodiment of the present invention. As shown in Fig. 4, the gimbal assembly includes an outer gimbal 313 and an inner gimbal 314 mounted in the outer gimbal 313. The outer gimbal 313 is coupled to the inner gimbal 314 by the fifth rotational joint 310'. Moreover, the outer gimbal 313 is rotatably coupled to the second translational arm 39' by the fourth rotational joint 310. In this case, the fifth rotational arm serves as the inner gimbal 314 of the gimbal assembly, and the fourth rotational arm as the outer gimbal 313 of the gimbal assembly.

With continued reference to Fig. 3, the instrument platform 3 further includes a housing 315 on which the interface 312 is provided. The interface 312 is preferred to be a cavity. The inner gimbal 314 drives the housing 315 to rotate together therewith by the interface 312, and the outer gimbal 313 is rotatably coupled to the inner gimbal 314. An axis of rotation X1 of the inner gimbal 314 relative to the outer gimbal 313 is perpendicular to an axis of rotation X2 of the outer gimbal 313 relative to the second translational arm 39', enabling yaw in two directions. Eventually, the instrument platform 3 is allowed to pitch (e.g., about the axis X1) and yaw (e.g., about the axis X2) relative to the second translational arm 39'. Coupled with the rotatability of the third rotational joint 38, orientational adjustability of the instrument assembly 40 in the base's coordinate system is enabled.

The housing 315 is disposed at a distal end thereof with a sleeve 311, which extends outwardly from the distal end of the housing 315 and is configured to be deployed at an incision made in the patient. The housing 315 may be either integral with or separate from the sleeve 311. In this embodiment, the housing 315 is provided therein with several instrument channels extending from a proximal end of the housing 315 to a distal end of the sleeve 311. The number of the instrument channels may be determined as required by an actual surgical procedure. In general cases, each instrument channel is provided therein with one surgical instrument 1 or one endoscope 2 detachably secured in the instrument channel. For example, during the assembly of the instrument assembly 40, the surgical instrument 1 or endoscope 2 may be detachably arranged on the housing 315 in such a manner that a proximal portion of the surgical instrument 1 extends into the housing 315 from the proximal end of the housing 315 and is accommodated therein, while a distal portion of the surgical instrument 1 extends into the housing 315 from the distal end of the housing 315 and is accommodated therein. Moreover, the distal portion of the surgical instrument 1 extends through and beyond the sleeve 311 into the patient's body for surgical operation.

Further, the instrument platform 3 comprises a tool axis, a plane of symmetry and a working plane. The working plane is perpendicular to the plane of symmetry and crosses axes of at least two of the instrument channels. The instrument channels are arranged in symmetry with respect to the plane of symmetry, and the tool axis is defined as the intersection of the plane of symmetry and the working plane. In actual surgical operation, a trocar is held at an incision made in the patient's skin, and the sleeve 311 is passed through the trocar into the patient's abdomen. Moreover, the distal end of the surgical instrument 1 or endoscope 2 is inserted through the trocar into the patient's body. However, in an actual surgical procedure, one, two or more surgical instruments 1 may be used, as determined by a surgeon according to the need of the procedure. Figs. 3 and 5 show an example in which two surgical instruments 1 are used, which may be identically structured. However, it will be appreciated that these surgical instruments 1 may differ from each other in some aspect. In particular, they may be equipped with different end effectors 6 having distinct functions. The present invention is not limited to any particular types of the end effectors, and a surgeon can select appropriate end effectors according to the need of an actual surgical procedure. For example, the end effectors may be selected as scissors, grasping forceps, a clamp, tweezers, other forceps, a resistive heater, an electric end effector such as a motor-driven tool, etc. Of course, the end effectors 6 may also be selected as other tools according to the surgeon's need, such as hooks or the like.

The following example where two surgical instruments 1 are used will be continued to explain how the surgical instrument platform 100 can easily allow multiple surgical instruments 1 to simultaneously approach the same target tissue without mutual interference between the surgical instruments 1. However, this should not be construed as limiting the present invention in any sense.

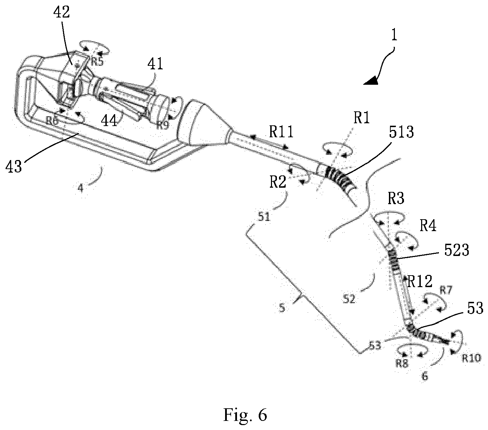

Fig. 6 is a schematic diagram showing the structure of one of the surgical instruments according to the first embodiment of the present invention. As shown in Fig. 6, in this embodiment, the surgical instrument 1 includes a manipulation end 4, a connection assembly 5 and an end effector 6. The connection assembly 5 includes a proximal first connecting structure 51 and a distal second connecting structure 52. A proximal end of the first connecting structure 51 is coupled to the manipulation end 4 and a distal end thereof is coupled to the instrument platform 3. A proximal end of the second connecting structure 52 is coupled to the instrument platform 3 and a distal end thereof is coupled to the end effector 6. Accordingly, a proximal portion of the surgical instrument 1 includes the manipulation end 4 and the first connecting structure 51, and a distal portion thereof includes the second connecting structure 52 and the end effector 6.

Additionally, the first connecting structure 51 includes a first flexible structure 513 with at least one rotational degree of freedom, which allows the first connecting structure 51 to be tilted from the tool axis. The second connecting structure 52 includes a second flexible structure 523 with at least one rotational degree of freedom, which allows the second connecting structure 52 to be tilted from the tool axis.

Further, the connection assembly 5 includes third flexible structures 53 arranged between the respective second connecting structures 52 and the respective end effectors 6. Each third flexible structure 53 has at least one rotational degree of freedom, which allows the respective end effector 6 to be tilted from the tool axis. That is, it can drive the end effector 6 to rotate. Thus, the distal portions of the surgical instruments 1 further include the respective third flexible structures 53.

Once the trocar is held stationary at the incision made in the patient, the instrument assembly 40 in an initial configuration is introduced into the patient's body through the trocar. In the initial configuration, as shown in Fig. 3, the two surgical instruments 1 comprise a shape resembling the letter "Y". That is, first flexible structure the manipulation ends 4 of the two surgical instruments 1 (i.e., the portions of the surgical instruments that remain outside of the patient's body) are oriented away from each other through the first flexible structures 513, while the portions that are inserted through the sleeve 311 into the patient's body (i.e., the portions of the surgical instruments that are deployed in the patient's body) are positioned adjacent each other and juxtaposed within in the working plane.

Specifically, in the initial configuration, the first flexible structures 513 of the surgical instruments 1 are configured to be each tilted from the tool axis at an angle, while the second flexible structures 523 are configured not to be tilted relative to the tool axis. That is, axes of the second connecting structures 52 are parallel to the tool axis. As a result, the distal portions of the surgical instruments 1 each comprises a linear shape and are parallel to the tool axis, while the proximal portions of the surgical instruments 1 are curved. Preferably, in the initial configuration, the third flexible structures 53 and even the end effectors 6 (if they have tilting degrees of freedom) are not tilted relative to the tool axis. As such, in each surgical instrument 1, the portion from the manipulation end 4 to the first flexible structure 513 is tilted from the tool axis, while the portion from the second flexible structure 523 to the end effector 6 substantially extends along a straight line parallel to the tool axis. That is, axes of the second flexible structure 523, the third flexible structure 53 and the end effector 6 are collinear. When each surgical instrument 1 is inserted in the trocar, the first flexible structure 513 and the second flexible structure 523 are positioned outside and inside the patient's body, respectively. This configuration (i.e., the initial configuration) facilitates not only the insertion of both surgical instruments 1 into the patient's body but also the removal of the instrument assembly 40 after the surgical procedure is completed. This enables easier assembly and disassembly of the instrument assembly 40 and facilitates subsequent controlled deployment of both surgical instruments 1 in the vicinity of the same target tissue without interference therebetween.

After the two surgical instruments 1 are inserted into the patient's body, referring to Fig. 5, they are deployed into a deployed configuration, where the proximal portions of the surgical instruments 1 are more curved than in the initial configuration, while the distal portions of the surgical instruments 1 each is a C-like shape oriented in the working plane. Specifically, the manipulation ends 4 of the two surgical instruments 1 are oriented more away from each other, while the sections of the two surgical instruments 1 deployed in the patient's body including, for example, the second flexible structures 523, the third flexible structures 53 and the end effectors 6 each comprises a C-like shape oriented in the working plane. Here, the C-like shape of each surgical instrument section shall not be interpreted narrowly as a circular arc; rather, it shall be interpreted broadly as a shape with portions oriented in different directions. For example, for each of the surgical instruments, the section deployed within the patient's body is first curved away from the tool axis by the second flexible structure 523 and then curved toward the tool axis by the third flexible structure 53. This facilitates approach of two or more end effectors 6 to the same target tissue without mutual crossing or rubbing of the surgical instruments 1 in case of simultaneous operation, thereby ensuring their normal use and avoiding secondary damage from being caused to the patient. Moreover, by suspending the instrument assembly 40 on the adjustment assembly 30, a surgeon can be saved from the trouble of handholding the instrument assembly 40 during a surgical procedure, enabling more time-saving and labor-saving surgical operation.

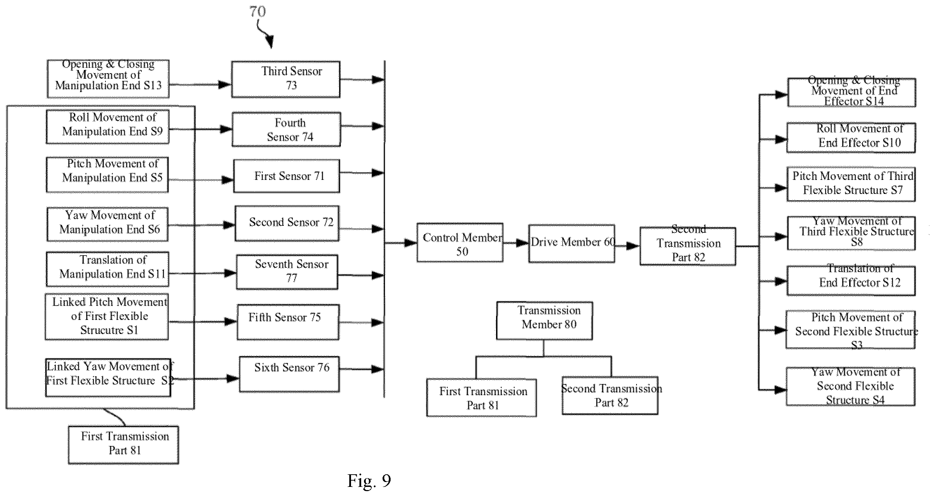

With continued reference to Fig. 6, in conjunction with Fig. 9, each surgical instrument 1 further includes a transmission member 80, a drive member 60, a sensor member 70 and a control member 50. Preferably, both the drive member 60 and the control member 50 are disposed in the instrument platform 3. The control member 50 is communicatively connected to both the drive member 60 and the sensor member 70. The sensor member 70 is configured to detect movement of the first connecting structure 51 and send the detected movement information of the first connecting structure 51 to the control member 50. The control member 50 is configured to control the drive member 60 to output power based on the detected movement information of first connecting structure 51 so that the drive member 60 can drive movement of the second connecting structure 52 through the transmission member 80. With this design, the connection assembly 5 can have improved movement accuracy.

The sensor member 70 is also configured to detect rotation of the first flexible structure 513 and send the detected rotation information to the control member 50, and the control member 50 is also configured to control the drive member 60 to output power based on the detected rotation information of the first flexible structure 513 so that the drive member 60 can drive the second flexible structure 523 by the transmission member 80 to oppositely rotate with the rotation of the first flexible structure 513. In this way, a linkage between the first flexible structure 513 and the second flexible structure 523 is established (for mirrored movement thereof with respect to the trocar). In one implementation, an angle of rotation of the second flexible structure 523 is configured to equate an rotation angle of the first flexible structure 513. In another implementation, the angle of rotation of the second flexible structure 523 is configured to be proportional to the angle of rotation of the first flexible structure 513.

In this embodiment, as shown in Fig. 6, each of the first flexible structure 513 and the second flexible structure 523 has two rotational degrees of freedom. For example, the first flexible structure 513 has a degree of freedom of rotation about a first axis R1 (i.e., pitch) and another degree of freedom of rotation about a second axis R2 (i.e., yaw), whilst the second flexible structure 523 has a degree of freedom of rotation about a third axis R3 (i.e., pitch) and another degree of freedom of rotation about a fourth axis R4 (i.e., yaw). Preferably, the third axis is parallel to the first axis, and the fourth axis is parallel to the second axis. More preferably, the first axis is perpendicular to the second axis. When the first flexible structure 513 is being driven to rotate about the first axis R1, the sensor member 70 detects the rotation of the first flexible structure 513 about the first axis R1 and sends this first detected rotation information to the control member 50. The control member 50 then controls the drive member 60 to output power based on the received first detected rotation information so that the drive member 60 can drive the second flexible structure 523 by the transmission member 80 to rotate about the third axis R3 in the opposite direction to that of the first flexible structure 513. Similarly, when the first flexible structure 513 is being driven to rotate about the second axis R2, the sensor member 70 detects the rotation of the first flexible structure 513 about the second axis R2 and sends this second detected rotation information to the control member 50. The control member 50controls the drive member 60 to output power based on the received second detected rotation information so that the drive member 60 can drive the second flexible structure 523 by the transmission member 80 to rotate about the fourth axis R4 in the opposite direction to that of the first flexible structure 513.

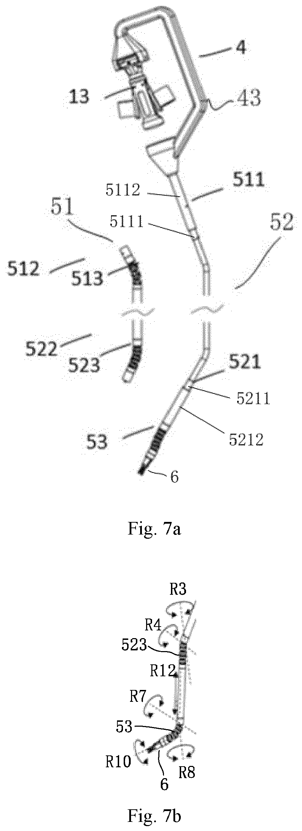

Further, as shown in Figs. 6, 7a and 7b, the first connecting structure 51 specifically includes a first outer tube 512 and a first inner tube 511, and the second connecting structure 52 includes a second outer tube 522 and a second inner tube 521. The first outer tube 512 nests over the first inner tube 511, and the second outer tube 522 nests over the second inner tube 521. That is, the first connecting structure 51 and the second connecting structure 52 are separate components each consisting of an inner tube and an outer tube nesting over the inner tube. More specifically, a proximal end of the first inner tube 511 is coupled to the manipulation end 4, and a distal end of the first inner tube 511 is a free end and is configured to be inserted in the first outer tube 512. A proximal end of the first outer tube 512 is a free end positioned outside the instrument platform 3, and a distal end of the first outer tube 512 is coupled to the instrument platform 3 (e.g., detachably or undetachably coupled to an instrument channel in the housing 315 of the instrument platform 3). A distal end of the second inner tube 521 is coupled to the end effector 6, preferably by the third flexible structure 53. A proximal end of the second inner tube 521 is a free end and is configured to be inserted in the second outer tube 522. A proximal end of the second outer tube 522 is coupled to the instrument platform 3 (e.g., detachably or undetachably coupled to an instrument channel in the housing 315 of the instrument platform 3, or to the sleeve 311), and a distal end of the second outer tube 522 is a free end disposed outside of the instrument platform 3. In addition, the first flexible structure 513 is provided on the first outer tube 512 and the second flexible structure 523 on the second outer tube 522. Optionally, the distal end of the first inner tube 511 of the first connecting structure 51 may extend into the instrument platform 3, for example, into an instrument channel in the instrument platform 3. Likewise, the proximal end of the second inner tube 521 of the second connecting structure 52 may extend into the instrument platform 3, for example, into an instrument channel.

More preferably, in the connection assembly 5, the first inner tube 511 is additionally capable of translation relative to the first outer tube 512 (including both distal advancement and proximal retraction). That is, the first inner tube 51 has a degree of freedom of translation along an axis R11 of the first outer tube 512, which enables advancement and retraction of the manipulation end 4 relative to the instrument platform 3. Moreover, the second inner tube 521 is additionally capable of translation relative to the second outer tube 522 (including both distal advancement and proximal retraction). That is, the second inner tube 521 has a degree of freedom of translation along an axis R12 of the second outer tube 522, which enables advancement and retraction of the end effector 6 relative to the instrument platform 3. In this case, movability of the first connecting structure 51 further includes translatability of the first inner tube 511, and similarly, movability of the second connecting structure 52 further includes translatability of the second inner tube 521. In practical use, advancement or retraction of the manipulation end 4 will cause synchronous advancement or retraction of the first inner tube 511 relative to the first outer tube 512. Accordingly, the sensor member 70 is configured to detect translation of the first inner tube 511 and/or the manipulation end 4 and obtain detected translation information, and the control member 50 is configured to control the drive member 60 to output power based on the detected translation information of the first inner tube 511 and/or the manipulation end 4 so that the drive member 60 can drive, by the transmission member 80, the second inner tube 521 to translate to cause the third flexible structure 53 and the end effector 6 to approach or move away from the target tissue. This can avoid falling off of the trocar and secondary damage such as subcutaneous edema due to repeated insertion and withdrawal of the surgical instrument 1 during the surgical procedure. Further, each of the first inner tube 511 and the second inner tube 521 is a hollow structure comprising a central channel running through its entire length, in which a part of the transmission member (e.g., a flexible shaft) may be accommodated. Furthermore, the transmission member may be provided within the housing 315 of the instrument platform 3 in order to make the surgical instrument compacter.

Additionally, the first inner tube 511 includes, joined together sequentially from the proximal end to the distal end thereof, a first proximal segment, a first intermediate segment and a first distal segment. The first proximal segment and the first distal segment are substantially rigid (i.e., they are rigid segments), and the first intermediate segment is substantially flexible (i.e., it is a flexible segment). In this embodiment, a "rigid" segment refers to a segment which will not experience bending and twisting deformations during its use (in surgery), while a "flexible" segment refers to a segment that is relatively more bendable during its use (in surgery). The first proximal segment is coupled to the manipulation end 4. In addition, the first outer tube 512 includes, joined together sequentially from the proximal end to the distal end thereof, a first proximal section, the first flexible structure 513 and a first distal section. The first distal section is coupled to the instrument platform 3. An aggregate axial length of the first proximal segment and the first intermediate segment in the first inner tube 511 is greater than an aggregate axial length of the first proximal section and the first flexible structure 513. The first distal segment of the first inner tube 511 is accommodated in the first distal section of the first outer tube 512, and the first distal segment may further extend into the instrument platform 3, e.g., into an instrument channel in the instrument platform 3.

As shown in Fig. 7a, for example, a proximal portion of the first proximal segment in the first inner tube 511 may be configured as a stepped rigid tube. In particular, it may include a first inner sub-tube 5111 and a second inner sub-tube 5112. An outer diameter of the first inner sub-tube 5111 is not greater than an inner diameter of the first proximal section in the first outer tube 512, thereby allowing direct accommodation of the first inner sub-tube 5111 in the first proximal section of the first outer tube 512. An outer diameter of the second inner sub-tube 5112 is greater than the inner diameter of the first proximal section. In this way, when the first outer tube 512 is held stationary and the first inner tube 511 is pushed distally, the second inner sub-tube 5112 will come into abutment against the first proximal section of the first outer tube 512, thereby limiting further distal movement of the first inner tube 511. In this case, an aggregate axial length of the first inner sub-tube 5111 and the first intermediate segment is greater than the aggregate axial length of the first proximal section and the first flexible structure 513.

Further, the second inner tube 521 includes, joined together sequentially from the proximal end to the distal end thereof, a second proximal segment, a second intermediate segment and a second distal segment. The second proximal segment and the second distal segment are substantially rigid, and the second intermediate segment is substantially flexible. In this embodiment, a "rigid" segment refers to a segment which will not experience bending and twisting deformations during its use (in surgery), while a "flexible" segment refers to a segment that is relatively more bendable during its use (in surgery). In addition, the second outer tube 522 includes, joined together sequentially from the proximal end to the distal end thereof, a second proximal section, the second flexible structure 523 and a second distal section. The second proximal portion is coupled to the instrument platform 3 or the sleeve 311. Optionally, the second proximal segment of the second inner tube 521 may extend into the instrument platform 3 and be coupled to the drive member disposed in the instrument platform 3. A distal end of the second distal segment of the second inner tube 521 may extend through the distal end of the second outer tube 522 and be coupled to the third flexible structure 53. Further, an aggregate axial length of the second intermediate segment and the second distal segment in the second inner tube 521 is greater than an aggregate axial length of the second flexible structure 523 and the second distal portion in the second outer tube 522.

Likewise, a distal portion of the second distal segment in the second inner tube 521 may be configured as a stepped rigid tube, which may in particular include a third inner sub-tube 5211 and a fourth inner sub-tube 5212. An outer diameter of the third inner sub-tube 5211 is not greater than an inner diameter of the second distal portion in the second outer tube 522, allowing direct accommodation of the third inner sub-tube 5211 in the second distal portion. An outer diameter of the fourth inner sub-tube 5212 is greater than the inner diameter of the second distal portion. In this way, when the second outer tube 522 is held stationary and the second inner tube 521 is retracted proximally, the fourth inner sub-tube 5212 will come into abutment against the second distal portion, limiting further proximal movement of the second inner tube 521. In this case, an aggregate axial length of the third inner sub-tube 5211 and the second intermediate segment is greater than the aggregate axial length of the second distal portion and the second flexible structure 523.

In an alternative embodiment, the connection assembly 5 may include one outer tube and one inner tube. In this case, the outer tube may function as both the aforementioned first and second outer tubes and the inner tube as both the aforementioned first and second inner tubes. The outer tube nests over the inner tube, and the inner tube is longer than the outer tube. The proximal end of the inner tube protrudes out of the outer tube and is coupled to the manipulation end 4. Moreover, the distal end of the inner tube protrudes out of the outer tube and is preferably coupled to the end effector 6 by the third flexible structure 53. Preferably, the inner tube is able to move relative to the outer tube. Further, the outer tube is secured to the instrument platform 3. For example, the outer tube may be detachably coupled to an instrument channel in the instrument platform 3. For example, the outer tube may be provided with a securing element, which secures the outer tube in the instrument channel in order to prevent undesirable movement of the surgical instrument within the instrument channel to avoid falling off of the trocar or other issues such as subcutaneous damage due to repeated insertion and withdrawal of the surgical instrument. Further, the first flexible structure 513 is disposed at a proximal end of the outer tube and the second flexible structure 523 at a distal end of the outer tube. Preferably, the distal end of the inner tube is coupled to the third flexible structure 53. In this case, likewise, the sensor member is configured to detect rotation of the first flexible structure 513 and obtain detected rotation information, and the control member 50 is configured to control the drive member 60 to output power based on the detected rotation information of the first flexible structure 513 so that the drive member 60 can drive, by the transmission member 80, the second flexible structure 523 to rotate in the opposite direction to that of the first flexible structure 513.

It would be appreciated that, according to the above embodiment, a linkage is established between the first flexible structure 513 and the second flexible structure 523 (for mirrored movement thereof) to enable insertion of two or more surgical instruments into the patient's body for interference-free simultaneous surgical operation. This reduces secondary damage that may be caused to the patient, and the surgical instruments can simultaneously approach the same target tissue to perform the surgical operation within an expanded working space, reducing surgical difficulties. In particular, the control member 50 controls, based on a rotational orientation of the first flexible structure 513, the drive member 60 to adjust a rotational orientation of the second flexible structure 523, enabling more precise movement of the second flexible structure 523 and additionally increasing surgical accuracy.

The sensor member 70 is further configured to detect movement of the manipulation end 4 and to send the detected movement information of the manipulation end 4 to the control member 50. The control member 50 is further configured to control the drive member 60 to output power based on the detected movement information of the manipulation end 4 so that the drive member 60 can drive, by the transmission member 80, the end effector 6 to effect desired movement of the end effector 6, which occurs in the same direction to that of the manipulation end 4.

With continued reference to Fig. 6, the manipulation end 4 includes a manipulator structure 41, a wrist structure 42 and a grip structure 43, which are connected together sequentially in this order. The manipulator structure 41 is provided on the wrist structure 42 and configured to drive the wrist structure 42 to move. The wrist structure 42 has at least one rotational degree of freedom. To this end, the wrist structure 42 is coupled to a proximal end of the grip structure 43 so as to be rotatable relative to the grip structure 43. The grip structure 43 is coupled to a proximal end of the connection assembly 5. More specifically, the grip structure 43 may be coupled to a proximal end of the first connecting structure 51. Preferably, the grip structure 43 is a curved structure. The present invention is not limited to any particular shape of the curved structure, and it may be designed ergonomically. Optionally, the grip structure 43 may be provided with a proximal mount coupled to the wrist structure 42 and a distal mount secured to the connection assembly 5. The securing may be accomplished by detachable or undetachable coupling.

In this embodiment, the wrist structure 42 has two rotational degrees of freedom respectively rotating about a fifth axis R5 and a sixth axis R6. Preferably, the fifth axis R5 is perpendicular to the sixth axis R6. The third flexible structure 53 has at least one rotational degree of freedom. For example, the third flexible structure may have two rotational degrees of freedom respectively rotating about a seventh axis R7 and an eighth axis R8. Preferably, the seventh axis R7 is perpendicular to the eighth axis R8.The fifth axis R5 is parallel to the seventh axis R7, and the sixth axis R6 is parallel to the eighth axis R8. When the wrist structure 42 is rotating about the fifth axis R5 under the drive of the manipulator structure 41, the sensor member 70 is configured to detect the rotation of the wrist structure 42 about the fifth axis R5 and send this third detected rotation information of the wrist structure 42 to the control member 50, which then controls the drive member 60 to output power based on the third detected rotation information of the wrist structure 42 so that the drive member 60 can drive, by the transmission member 80, the third flexible structure 53 to rotate about the seventh axis R7 in the same direction as that of the wrist structure 42. Similarly, when the wrist structure 42 is rotating about the sixth axis R6 under the drive of the manipulator structure 41, the sensor member 70 is configured to detect the rotation of the wrist structure 42 about the sixth axis R6 and send this fourth detected rotation information of the wrist structure 42 to the control member 50, which then controls the drive member 60 to output power based on the received fourth detected rotation information of the wrist structure 42 so that the drive member 60 can drive, by the transmission member 80, the third flexible structure 53 to rotate about the eighth axis R8 in the same direction as that of the wrist structure 42. Finally, the third flexible structure 53 is able to drive the end effector 6 to pitch and yaw.

According to the above embodiment, rotation of the wrist structure 42 and the third flexible structure 53 in the same direction enables the manipulation end 4 and the end effector 6 to rotate in the same direction (including pitch and yaw). This facilitates simultaneous approach of two or more surgical instruments to the same target tissue in the patient's body to perform the surgical operation, thereby additionally reducing surgical difficulties. Moreover, the control member controls, based on a rotational orientation of the manipulation end 4, the drive member to adjust a rotational orientation of the end effector 6, enabling more precise movement of the end effector 6 and additionally increasing surgical accuracy.

With continued reference to Fig. 6, the manipulator structure 41 has a roll degree of freedom about a ninth axis R9, and correspondingly, the end effector 6 has a roll degree of freedom about a tenth axis R10. Specifically, the manipulator structure 41 is rotatably disposed on the wrist structure 42. Correspondingly, the end effector 6 is rotatably coupled to the third flexible structure 53. In practice, when the manipulator structure 41 is rolling about the ninth axis R9, the sensor member 70 is configured to detect the rolling of the manipulator structure 41 and send the detected rolling information of the manipulator structure 41 to the control member 50, which then controls the drive member 60 to output power based on the detected rolling information of the manipulator structure 41 so that the drive member 60 can drive, by the transmission member 80, the end effector 6 to roll in the same direction as that of the manipulator structure 41. A roll angle of the end effector 6 may be configured to be equal or proportional to a roll angle of the manipulator structure 41.

The manipulator structure 41 may further have a degree of freedom of axial translation, and correspondingly, the end effector 6 may also have a degree of freedom of axial translation. In this case, the manipulator structure 41 is translatably disposed on the wrist structure 42. According to this embodiment, when the manipulator structure 41 is translating, the sensor member 70 is configured to detect the translation of the manipulator structure 41 and send the detected translation information of the manipulator structure 41 to the control member 50, which then controls the drive member 60 to output power based on the detected translation information of the manipulator structure 41 so that the drive member 60 can drive, by the transmission member 80, the end effector 6 to translate in the same direction as that of the manipulator structure 41. Specifically, as noted above, the first inner tube 511 is proximally coupled to the manipulator structure 41. The manipulator structure 41 is configured to drive the first inner tube 511 to translate with the manipulator structure along an eleventh axis R11. Therefore, once the first inner tube 511 is detected to be translating, it can be determined that the manipulator structure 41 is translating. In practice, the sensor member 70 may be configured to detect translation of either or both of the first inner tube 511 and the manipulator structure 41, without limiting the present invention. Based on the detected translation information of the first inner tube 511 and/or the manipulator structure 41, the control member 50 controls the drive member 60 to output power so that the drive member 60 can drive, by the transmission member 80, the second inner tube 521 and hence the end effector 6 to translate along a twelfth axis R12.

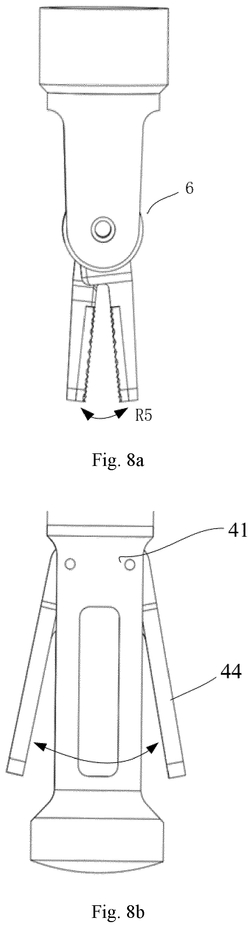

For different end effectors 6, the surgical instrument 1 may be configured with different degrees of freedom. For example, the end effector 6 may have one or two tool arms. For example, as shown in Fig. 8a, the end effector 6 may be a clamp having two tool arms capable of performing clamping operations. By the transmission member 80, the drive member 60 can cause opening or closing of the end effector 6. As shown in Fig. 8b, the manipulation end 4 further includes an opening and closing control structure 44 provided on the manipulator structure 41. In practice, when the opening and closing control structure 44 is making opening and closing movement, the sensor member 70 is configured to detect the opening and closing movement of the opening and closing control structure 44 and send the detected opening and closing movement information of the opening and closing control structure 44 to the control member 50, which then controls the drive member 60 to output power based on the received detected opening and closing movement information so that the drive member 60 can drive, by the transmission member 80, opening and closing movement of the tool arms of the end effector 6, the end effector 6 occurs in the same manner (or pattern) as the opening and closing movement of the opening and closing control structure 44. That is, opening of the opening and closing control structure 44 results in opening of the tool arms of the end effector 6, and closing of the opening and closing control structure 44 causes closing of the tool arms of the end effector 6. The manipulator structure 41 may include, for example, a manipulation rod (not labeled, see Fig. 8b). In one embodiment, the opening and closing control structure 44 includes a first pivotal arm and a second pivotal arm. Both pivotal arms are pivotally coupled at one end to the manipulation rod of the manipulator structure 41 and extend outwardly at the other end. In another embodiment, the opening and closing control structure 44 may also include only one pivotal arm.

With additional reference to Fig. 9, in this embodiment, the sensor member 70 may include a first sensor 71 and a second sensor 72. The first sensor 71 is configured to detect pitch movement S5 of the wrist structure 42, and the second sensor 72 is configured to detect yaw movement S6 of the wrist structure 42. The first sensor 71 and the second sensor 72 may be angle sensors such as shaft encoder discs or rotary encoders. The sensor member 70 may further include a third sensor 73 configured to detect opening and closing movement S13 of the opening and closing control structure 44. The sensor member 70 may further include a fourth sensor 74 configured to detect roll movement S9 of the manipulator structure 41. The third sensor 73 may be a Hall sensor, which is disposed between the pivotal arms of the opening and closing control structure 44 and the manipulator structure 41 and is configured to detect pivotal movement of the pivotal arms of the opening and closing control structure 44 relative to the manipulator structure 41. In other embodiments, at least one third sensor 73 each implemented as a shaft encoder disc may be disposed on a shaft at proximal ends of the pivotal arms of the opening and closing control structure 44 and configured to detect the pivotal movement of the pivotal arms. The fourth sensor 74 may also be an angle sensor such as a shaft encoder disc or a rotary encoder, which is configured to detect roll movement of the manipulator structure 41 about its own axis. The sensor member 70 may further include a fifth sensor 75 and a sixth sensor 76. The fifth sensor 75 is configured to detect pitch movement S 1 of the first flexible structure 513, and the sixth sensor 76 is configured to detect yaw movement S2 of the first flexible structure 513. Both the fifth sensor 75 and the sixth sensor 76 may be angle sensors. The sensor member 70 may further include a seventh sensor 77 configured to detect translational movement S11 of the manipulation end 4 and hence of the first inner tube 511. The seventh sensor 77 may be selected as a linear sensor.

Any of the above sensors sends the movement information that it detects to the control member 50, the control member 50 then controls the drive member 60 to output power based on the movement information. In this way, the drive member 60 can control, by of the transmission member 80, the end effector 6, the third flexible structure 53, the second flexible structure 523 and the second inner tube 521 to move in a corresponding way. For example, based on the detected information from the third sensor 73, the drive member 60 can control the end effector 6 by the transmission member 80 to make opening and closing movement S 14 corresponding to opening and closing movement S 13 of the manipulation end 4. As another example, based on the detected information from the fourth sensor 74, the drive member 60 can control the end effector 6 by the transmission member 80 to make roll movement S 10 corresponding to that of the manipulation end 4. As yet another example, based on the detected information from the seventh sensor 77, the drive member 60 can control the end effector 6 by the transmission member 80 to make corresponding translational movement S12. As still yet another example, based on the detected information from the first sensor 71 and the second sensor 72, the drive member 60 can control the third flexible structure 53 by the transmission member 80 to make pitch S7 and yaw S8 movement corresponding to that of the wrist structure 42. As a result, driven by the third flexible structure 53, the end effector 6 will make pitch and yaw movement in the same directions as that of the manipulation end 4. Likewise, based on the detected information from the fifth sensor 75 and the sixth sensor 76, the drive member 60 can control the second flexible structure 523 by the transmission member 80 to make opposite pitch S3 and yaw S4 movement to that of the first flexible structure 513.

With additional reference to Fig. 9, the transmission member 80 includes a first transmission part 81 and a second transmission part 82. The first transmission part 81 is configured to convert movement of the first connecting structure 51 to a movement detectable by the sensor member 70. The second transmission part 82 is coupled to the drive member 60 so as to be able to drive movement of the second connecting structure 52. The first transmission part 81 is also configured to convert movement of the manipulation end 4, the first flexible structure 513 and the first inner tube 511 to a movement detectable by the sensor. The second transmission part 82 is configured to drive the third flexible structure 53, the end effector 6, the second inner tube 521 and the second flexible structure 523 to make corresponding movement.

For the pitch and yaw movement of the first flexible structure 513, the first transmission part 81 may include a first cable transmission assembly and a second cable transmission assembly. The first cable transmission assembly includes a first transmission cable and a first cable pulley, and is configured to use the first transmission cable to convert the pitch movement of the first flexible structure 513 about the first axis R1 into rotational movement of the first cable pulley. The second cable transmission assembly includes a second transmission cable and a second cable pulley, and is configured to use the second transmission cable to convert the yaw movement of the first flexible structure 513 about the second axis R2 into rotational movement of the second cable pulley. Specifically, the proximal end of the first transmission cable in the first cable transmission assembly is coupled to the first flexible structure 513, and the distal end of the first transmission cable is wound through the first cable pulley and then coupled to the first flexible structure 513, thereby enabling the first cable pulley in the first cable transmission assembly to follow the pitch movement of the first flexible structure 513 about the first axis R1 to make the rotational movement. The proximal end of the second transmission cable in the second cable transmission assembly is coupled also to the first flexible structure 513, and the distal end of the second transmission cable is wound through the second cable pulley and then coupled to the first flexible structure 513, thereby enabling the second cable pulley in the second cable transmission assembly to follow the yaw movement of the first flexible structure 513 about the second axis R2 to make the rotational movement. Additionally, the fifth sensor 75 may be provided on the first cable pulley and the sixth sensor 76 on the second cable pulley. The fifth sensor 75 detects the rotational movement of the first cable pulley and sends the detected rotation information of the first cable pulley to the control member 50, the control member 50 then determines a condition of rotation (or rotational orientation) of the first flexible structure 513 about the first axis R1 from the detected rotation information of the first cable pulley and controls the drive member 60 to output power based on the detected rotation information of the first cable pulley to drive the second flexible structure 523 to rotate about the third axis R3. Similarly, the sixth sensor 76 detects the rotational movement of the second cable pulley and sends the detected rotation information of the second cable pulley to the control member 50, the control member 50 then determines a condition of rotation of the first flexible structure 513 about the second axis R2 from the detected rotation information of the second cable pulley and controls the drive member 60 to output power based on the detected rotation information of the second cable pulley to drive the second flexible structure 523 to rotate about the fourth axis R4.

Likewise, for the pitch and yaw movement of the wrist structure 42, the first transmission part 81 may further include a third cable transmission assembly and a fourth cable transmission assembly. The third cable transmission assembly includes a third transmission cable and a third cable pulley and is configured to use the third transmission cable to convert the pitch movement of the wrist structure 42 about the fifth axis R5 into rotational movement of the third cable pulley. The fourth cable transmission assembly includes a fourth transmission cable and a fourth cable pulley and is configured to use the fourth transmission cable to convert the yaw movement of the wrist structure 42 about the sixth axis R6 into rotational movement of the fourth cable pulley. Specifically, the proximal end of the third transmission cable in the third cable transmission assembly is coupled to the wrist structure 42, and the distal end of the third transmission cable is wound through the third cable pulley and then coupled to the wrist structure 42, thereby enabling the third cable pulley in the third cable transmission assembly to follow the pitch movement of the wrist structure 42 about the fifth axis R5 to make the rotational movement. The proximal end of the fourth transmission cable in the fourth cable transmission assembly is coupled also to the wrist structure 42, and the distal end of the fourth transmission cable is wound through the fourth cable pulley and then coupled to the wrist structure 42, thereby enabling the fourth cable pulley in the fourth cable transmission assembly to follow the yaw movement of the wrist structure 42 about the sixth axis R6 to make the rotational movement. Additionally, the first sensor 71 may be provided on the third cable pulley and the second sensor 72 on the fourth cable pulley. The first sensor 71 detects the rotational movement of the third cable pulley and sends the detected rotation information of the third cable pulley to the control member 50, the control member 50 then determines a condition of rotation (or rotational orientation) of the wrist structure 42 about the fifth axis R5 from the detected rotation information of the third cable pulley and controls the drive member 60 to output power based on the detected rotation information of the third cable pulley to drive the third flexible structure 53 to rotate about the seventh axis R7. Similarly, the second sensor 72 detects the rotational movement of the fourth cable pulley and sends the detected rotation information of the fourth cable pulley to the control member 50, the control member 50 then determines a condition of rotation of the wrist structure 42 about the sixth axis R6 from the detected rotation information of the fourth cable pulley and controls the drive member 60 to output power based on the detected rotation information of the fourth cable pulley to drive the third flexible structure 53 to rotate about the eighth axis R8. Preferably, all the aforementioned cable pulleys are mounted in the housing 315 of the instrument platform 3.

Further, for the translational movement of the manipulator structure 41 along an axis of the connection assembly 5, the first transmission part 81 may include a first rack and pinion transmission mechanism including a first rack and a first pinion in meshing engagement with the first rack. The first rack is coupled to the first inner tube 511, e.g., to the first distal segment of the first inner tube 511. In this way, the translational movement of the first inner tube 511 can be converted into rotational movement of the first pinion. The seventh sensor 77 may be provided on the first pinion and configured to detect the rotational movement thereof. Thus, a condition of translation of the first inner tube 511 and hence of the manipulator structure 41 can be determined from the detected rotation information of the first pinion. Accordingly, the control member 50 can control the drive member 60 to output power based on the detected rotation information of the first pinion to drive the second inner tube 521 to translate. Preferably, the first rack and pinion transmission mechanism is provided in the housing 315 of the instrument platform 3.

Furthermore, for the roll movement of the manipulator structure 41, the first transmission part 81 may further include a first shaft assembly including a first flexible shaft and a first rotary wheel. The proximal end of the first flexible shaft is coupled to the manipulator structure 41 and the distal end of first flexible shaft is coupled to the first rotary wheel, enabling the first rotary wheel to follow the roll movement of the manipulator structure 41 to make rotational movement. The fourth sensor 74 may be provided on the first rotary wheel and configured to detect the rotational movement of the first rotary wheel. In this way, a condition of rolling of the manipulator structure 41 can be determined from the detected rotation information of the first rotary wheel. Accordingly, the control member 50 can control the drive member 60 to output power based on the detected rotation information of the first rotary wheel so that, by the second transmission part 82, the drive member 60 can drive the end effector 6 to roll. Preferably, the first rotary wheel is provided in the housing 315 of the instrument platform 3. Further, the first flexible shaft may pass through the wrist structure 42, the grip structure 43 and the first connecting structure 51 into the housing 315.

Preferably, the third sensor 73 can directly detect, without conversion, the opening and closing movement of the opening and closing control structure 44. For example, the third sensor 73 may be disposed on a shaft of the opening and closing control structure 44 and configured to detect the opening and closing movement of the pivotal arms in the opening and closing control structure 44.

Further, both the control member 50 and the drive member 60 are provided in the housing 315 of the instrument platform 3. In particular, the drive member 60 may include several motors for driving the end effector 6, the third flexible structure 53, the second flexible structure 523 and the second inner tube 521 to make corresponding movement by the second transmission part 82. The control member 50 is communicatively connected to both the sensor member 70 and the drive member 60, more precisely, mainly to electrical elements in these components, so as to control automatic operation of moving parts in these components. This embodiment is not limited to any particular form of the control member 50, and the control member 50 may be implemented by hardware that performs logical operations, such as a single-chip microcomputer device, a microprocessor, a programmable logic controller (PLC) or a field-programmable gate array (FPGA), or by a software program, functional module, function, object library or dynamic-link library that performs the above functions on the basis of hardware, or by a combination of both. Those skilled in the art would know how to achieve the communication between the control member 50 and the other components based on the teachings disclosed herein.

Additionally, the second transmission part 82 may include a fifth cable transmission assembly and a sixth cable transmission assembly. The fifth cable transmission assembly is configured to drive the second flexible structure 523 to make the pitch movement, and the sixth cable transmission assembly is configured to drive the second flexible structure 523 to make the yaw movement. Specifically, the fifth cable transmission assembly may include at least two fifth transmission cables, the proximal ends of the two fifth transmission cables are both coupled to a first motor (serving as a first drive component) and the distal ends of the two fifth transmission cables are both secured to the second flexible structure 523. With this arrangement, the first motor can drive the second flexible structure 523 to make the pitch movement (i.e., the rotational movement about the third axis) by using the two fifth transmission cables. The sixth cable transmission assembly may include at least two sixth transmission cables, the proximal ends of the two sixth transmission cables are both coupled to a second motor (serving as a second drive component) and the distal ends of the two sixth transmission cables are both secured to the second flexible structure 523. With this arrangement, the second motor can drive the second flexible structure 523 to make the yaw movement (i.e., the rotational movement about the third axis) by using the two sixth transmission cables.

The second transmission part 82 may further include a seventh cable transmission assembly and an eighth cable transmission assembly The seventh cable transmission assembly is configured to drive the third flexible structure 53 to make the pitch movement, and the eighth cable transmission assembly is configured to drive the third flexible structure 53 to make the yaw movement. Specifically, with similarity to the fifth and sixth cable transmission assemblies, the seventh cable transmission assembly may include at least two seventh transmission cables, the proximal ends of the two seventh transmission cables are both coupled to a third motor (serving as a third drive component) and the distal ends of the two seventh transmission cables are both passed through the second flexible structure 523 and secured to the third flexible structure 53. With this arrangement, the third motor can drive the third flexible structure 53 to make the pitch movement (i.e., the rotational movement about the seventh axis) by using the two seventh transmission cables. The eighth cable transmission assembly may include at least two eighth transmission cables, the proximal ends of the two eighth transmission cables are both coupled to a fourth motor, and the distal ends of the two eighth transmission cables are both distally passed through the second flexible structure 523 and secured to the third flexible structure 53. With this arrangement, the fourth motor can drive the third flexible structure 53 to make the yaw movement (i.e., the rotational movement about the eighth axis) by using the two eighth transmission cables. Further, each of the first, second, third and fourth motors may be provided with a cable pulley at an output thereof, through which the specific motor is coupled to the second transmission part 82.

The second transmission part 82 may further include a second rack and pinion transmission mechanism including a second pinion and a second rack in meshing engagement with the second pinion. The second pinion may be coupled to a fifth motor (serving as a fifth drive component) and the second rack to the second inner tube 521, e.g., to the second proximal segment of the second inner tube 521. With this arrangement, using the second rack and pinion transmission mechanism, the fifth motor can drive the second inner tube 521 and hence the end effector 6 to make the translational movement.

The second transmission part 82 may further include a second shaft assembly including a second flexible shaft, a proximal end of the second flexible shaft is coupled to a sixth motor (serving as a sixth drive component) and a distal end of the second flexible shaft is coupled to the end effector 6. In practice, when the manipulator structure 41 is making the roll movement about the ninth axis R9, the fourth sensor 74 detects this roll movement of the manipulator structure 41 and sends the detected rolling information of the manipulator structure 41 to the control member 50, the control member 50 then controls the drive member 60 based on the detected rolling information of the manipulator structure 41 to transmit the rotational movement to the end effector 6 through the second flexible shaft, enabling the end effector 6 to make the roll movement about the tenth axis R10. In addition, the end effector 6 may include a proximal effector mount, and the distal end of the second flexible shaft may be coupled to the proximal effector mount, in order to enable the proximal effector mount to make roll movement about the tenth axis R10. Further, the proximal effector mount may be rotatably coupled to the third flexible structure 53. Preferably, the first and second flexible shafts may be each made of a material capable of torque transmission, such as a tungsten or nickel-titanium wire.

The second transmission part 82 may further include a first adapter, a flexible transmission mechanism for opening and closing movement and a second adapter, which are coupled together sequentially in this order. The first adapter is coupled to a seventh motor (serving as a seventh drive component) and configured to convert the rotational movement of the seventh motor into axial movement of the flexible transmission mechanism. The second adapter is coupled to the tool arms of the end effector and configured to convert the axial movement of the flexible transmission mechanism into the opening and closing movement of the tool arms. Moreover, the flexible transmission mechanism, the first adapter and the second adapter are configured to cause the opening and closing control structure to move in the same manner as the tool arms of the end effector. In particular, the flexible transmission mechanism may be implemented as a transmission cable or flexible shaft capable of torque transmission. When the opening and closing control structure 44 is making the opening and closing movement, the third sensor 73 detects the opening and closing movement of the opening and closing control structure 44 and sends the detected opening and closing information of the opening and closing control structure 44 to the control member 50, the control member then controls the drive member 60 based on the detected opening and closing information of the opening and closing control structure 44 to drive the end effector 6 to make the opening and closing movement by the first adapter, the flexible transmission mechanism and the second adapter. In a preferred embodiment, in order to allow a surgeon to more efficiently manipulate the pivotal arms to open and close the arms with increased comfort, the two pivotal arms of the opening and closing control structure 44 may be provided thereon with finger cuffs for insertion of the surgeon's fingers therein.

It is to be noted that each of the transmission cables in this embodiment may be made of a material capable of torque transmission, such as a tungsten or nickel-titanium wire, and that all the motors are integrated in the housing 315 of the instrument platform 3.

Preferably, the surgical instrument further includes a locking mechanism such as a locking button, which, when pressed by an operator, can be controlled by the control member 50 to maintain the surgical instrument 1 in a deployed configuration at a desired angle. Additionally, the aforementioned yaw movement of each of the first and second flexible structures is preferably lockable by the locking mechanism optionally by locking the corresponding cable transmission assembly or flexible element. Further, the aforementioned yaw movement of each of the manipulator structure and the third flexible structure 53 is preferably lockable by the locking mechanism optionally by locking the corresponding cable transmission assembly, the manipulator structure, the corresponding flexible element or the like. Preferably, the transmission member 80 further includes a locking mechanism for locking the roll movement of the end effector, for example, by locking the corresponding flexible shaft. The locking mechanism of the surgical instrument may also be able to lock its opening and closing movement, for example, by locking the opening and closing movement of the opening and closing control structure, the axial movement of the flexible transmission mechanism, the opening and closing movement of the end effector, or the like.

With continued reference to Figs. 5 and 6, preferably, the second axis R2 is perpendicular to the working plane of the instrument assembly 40, and corresponding, the fourth axis R4 is also perpendicular to the working plane. Preferably, the fifth axis R5 is perpendicular to the working plane, and corresponding, the seventh axis R7 is also perpendicular to the working plane.

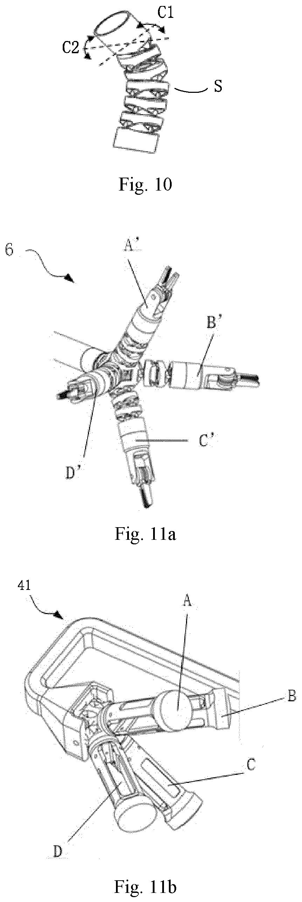

Further, the present invention is not limited to any particular form of any of the aforementioned flexible elements, as long as it can make yaw movement in two directions (which are preferred to be perpendicular to each other). In a preferred embodiment, as shown in Fig. 10, each of the flexible elements is implemented as a snake-arm robotic joint S having two yaw degrees of freedom about mutually perpendicular axes C1 and C2. Likewise, the present invention is not limited to any particular form of the wrist structure 42, as long as it can make yaw movement in two directions (which are preferred to be perpendicular to each other). Preferably, wrist structure 42 is a gimbal assembly, a spherical hinge or a snake-arm robotic hinge.

Further, as shown in Figs. 11a and 11b, with the above arrangement, the end effector 6 can rotate in the same direction and orientation as the manipulator structure 41. In response to the manipulator structure 41 switching between different configurations A, B, C and D as shown in Fig. 11b, the end effector 6 can switch between corresponding configurations A', B', C' and D' as shown in Fig. 11a, enabling the manipulator structure 41 and the end effector 6 to operate exactly in the same fashion. Moreover, under the control of the drive member 60, the third flexible structure 53 can operate with two degrees of freedom to drive the end effector 6 to precisely move toward or away from a target tissue site, as desired by a surgical procedure.

More preferably, the second cable transmission assembly may further include an elastic element, such as a compression spring for compensating for curvature of the second cable transmission assembly and thereby maintaining the surgical instrument 1 in a desired deployed configuration. In order to switch the surgical instrument 1 from the initial configuration to a desired deployed configuration, angles of the first flexible structure 513 and the second flexible structure 523 may be adjusted. Through providing the elastic element, a condition of the transmission cable in the second cable transmission assembly may be adjusted to maintain the surgical instrument 1 in the desired deployed configuration with a desirable orientation of the end effector. More specifically, the transmission cable in the second cable transmission assembly may be at least partially coupled to the compression spring in order to enable the curvature compensation of the transmission cable.

In summary, in the surgical instrument platform provided in embodiments of the present invention, movement of the connection assembly is controlled by the drive member to enable more precise movement control of the end effector and more convenient surgical operation with increased comfort. In particular, in the surgical instrument platform, the linkage established between the first and second flexible structures allows interference-free, simultaneous operation of two or more surgical instruments 1 both or all inserted into a patient's body, which reduce secondary damage that may be caused to the patient. Moreover, these surgical instruments 1 are enabled to simultaneously approach the same target tissue in an expanded working space, thereby reducing surgical difficulties. Additionally, by suspending the instrument assembly on the adjustment assembly, a surgeon can be saved from the trouble of handholding the instrument assembly during surgery, enabling more time-saving, more labor-saving, more precise and more reliable surgical operation. Further, the present invention is not limited to any general shape of the two surgical instruments in the initial configuration, and either a Y-shaped symmetrical shape or an asymmetrical structure is possible.

The description presented above is merely that of some preferred embodiments of the present invention and does not limit the scope thereof in any sense. Any and all changes and modifications made by those of ordinary skill in the art based on the above teachings fall within the scope as defined in the appended claims.

## Claims

1. A surgical instrument platform, comprising:
a base;
an adjustment assembly disposed on the base and comprising a mechanical arm; and
an instrument assembly comprising a surgical instrument and an instrument platform, wherein the surgical instrument is detachably disposed on the instrument platform, and wherein the instrument platform is coupled to an end of the mechanical arm so as to enable the mechanical arm to adjust a position and an orientation of the instrument assembly,
wherein: the surgical instrument comprises a manipulation end, a connection assembly and an end effector, which are connected sequentially from a proximal end to a distal end of the surgical instrument; the connection assembly comprises a proximal first connecting structure and a distal second connecting structure; a proximal end of the first connecting structure is coupled to the manipulation end, and a distal end of the first connecting structure is coupled to the instrument platform; a distal end of the second connecting structure is coupled to the end effector, and a proximal end of the second connecting structure is coupled to the instrument platform; and
wherein: the surgical instrument further comprises a sensor member, a drive member, a transmission member and a control member; the sensor member is configured to detect a movement of the first connecting structure and to send a detected movement information of the first connecting structure to the control member; the control member is configured to control the drive member to output a power based on the detected movement information of the first connecting structure; and the drive member is configured to drive a movement of the second connecting structure through the transmission member.

2. The surgical instrument platform of claim 1, wherein the first connecting structure comprises a first flexible structure, and wherein the second connecting structure comprises a second flexible structure, each of the first and second flexible structures having at least one rotational degree of freedom,
wherein: the sensor member is further configured to detect a rotation of the first flexible structure and to send a detected rotation information to the control member; the control member is further configured to control the drive member to output a power based on the detected rotation information of the first flexible structure; and the drive member is further configured to drive, through the transmission member, the second flexible structure to follow the rotation of the first flexible structure to rotate in an opposite direction.

3. The surgical instrument platform of claim 2, wherein the connection assembly further comprises a third flexible structure, wherein the third flexible structure is disposed between the second connecting structure and the end effector and has at least one rotational degree of freedom for driving the end effector to rotate, and wherein the manipulation end comprises a manipulator structure, a wrist structure and a grip structure that are sequentially connected, wherein the manipulator structure is disposed on the wrist structure and is configured to drive the wrist structure to move, wherein the grip structure is coupled to the proximal end of the first connecting structure, the wrist structure having at least one rotational degree of freedom,
wherein: the sensor member is configured to detect a rotation of the wrist structure and to send a detected rotation information to the control member; the control member is configured to control the drive member to output a power based on the detected rotation information of the wrist structure; and the drive member is configured to drive, through the transmission member, the third flexible structure to follow the rotation of the wrist structure to rotate in an opposite direction.

4. The surgical instrument platform of claim 2, wherein the first connecting structure comprises a first outer tube and a first inner tube, wherein the second connecting structure comprises a second outer tube and a second inner tube, the first outer tube nesting on the first inner tube, the second outer tube nesting on the second inner tube,
wherein: a proximal end of the first inner tube is coupled to the manipulation end; a distal end of the first inner tube is a free end and is configured to be inserted into the first outer tube; a proximal end of the first outer tube is a free end and is positioned outside of the instrument platform; and a distal end of the first outer tube is coupled to the instrument platform,
wherein: a distal end of the second inner tube is coupled to the end effector; a proximal end of the second inner tube is a free end and is configured to be inserted into the second outer tube; a proximal end of the second outer tube is coupled to the instrument platform; and a distal end of the second outer tube is a free end and is positioned outside of the instrument platform,
wherein: the first outer tube is provided therein with the first flexible structure; and the second outer tube is provided therein with the second flexible structure.

5. The surgical instrument platform of claim 4, wherein the distal end of the second inner tube is coupled to the end effector by a third flexible structure that has at least one rotational degree of freedom; and/or each of the distal end of the first inner tube and the proximal end of the second inner tube extends into the instrument platform.

6. The surgical instrument platform of claim 2 or 3, wherein the first flexible structure has two rotational degrees of freedom respectively about a first axis and a second axis, and
wherein the second flexible structure has two rotational degrees of freedom respectively about a third axis and a fourth axis,
wherein: the third axis is parallel to the first axis; the fourth axis is parallel to the second axis; and the first axis is perpendicular to the second axis,
wherein when the first flexible structure is being driven to rotate about the first axis, the sensor member is configured to detect the rotation of the first flexible structure about the first axis and to send a first detected rotation information to the control member; the control member is configured to control the drive member to output a power based on the first detected rotation information; and the drive member is configured to drive, through the transmission member, the second flexible structure to rotate about the third axis in an opposite direction to the rotation of the first flexible structure, and
wherein: when the first flexible structure is being driven to rotate about the second axis, the sensor member is configured to detect the rotation of the first flexible structure about the second axis and send a second detected rotation information to the control member; the control member is configured to control the drive member to output power based on the second detected rotation information; and the drive member is configured to drive, through the transmission member, the second flexible structure to rotate about the fourth axis in an opposite direction to the rotation of the first flexible structure.

7. The surgical instrument platform of claim 4, wherein: the first inner tube is able to translate with respect to the first outer tube; and the second inner tube is able to translate with respect to the second outer tube,
wherein: the sensor member is further configured to detect a translation of the first inner tube and/or the manipulation end and to send a detected translation information of the first inner tube and/or the manipulation end to the control member; the control member is configured to control the drive member to output a power based on a detected translation information of the first inner tube and/or the manipulation end; and the drive member is configured to drive the second inner tube to translate through the transmission member.

8. The surgical instrument platform of claim 7, wherein the first inner tube comprises a first proximal segment, a first intermediate segment and a first distal segment, which are connected sequentially from a proximal end to a distal end of the first inner tube, wherein the first proximal segment is coupled to the manipulation end, wherein each of the first proximal segment and the first distal segment is implemented as a rigid segment, the first intermediate segment implemented as a flexible segment, and wherein the first outer tube comprises a first proximal section, the first flexible structure and a first distal section, which are connected sequentially from a proximal end to a distal end of the first outer tube, the first distal section coupled to the instrument platform,
wherein an aggregate axial length of the first proximal segment and the first intermediate segment is greater than an aggregate axial length of the first proximal section and the first flexible structure, and wherein the first distal segment is accommodated in the first distal section and extends into the instrument platform.

9. The surgical instrument platform of claim 8, wherein a proximal portion of the first proximal segment comprises a first inner sub-tube and a second inner sub-tube, wherein the first inner sub-tube has an outer diameter that is not greater than an inner diameter of the first proximal section, wherein the second inner sub-tube has an outer diameter that is greater than the inner diameter of the first proximal section, wherein an aggregate axial length of the first inner sub-tube and the first intermediate segment is greater than the aggregate axial length of the first proximal section and the first flexible structure.

10. The surgical instrument platform of claim 7, wherein the second inner tube comprises a second proximal segment, a second intermediate segment and a second distal segment, which are connected sequentially from a proximal end to a distal end of the second inner tube, wherein each of the second proximal segment and the second distal segment is implemented as a rigid segment, the second intermediate segment implemented as a flexible segment, wherein the second distal segment is coupled to the end effector, and wherein the second proximal segment extends into the instrument platform and is coupled to the drive member, and
wherein the second outer tube comprises a second proximal section, the second flexible structure and a second distal section, which are connected sequentially from a proximal end to a distal end of the second outer tube, wherein the second proximal section is coupled to the instrument platform, wherein an aggregate axial length of the second intermediate segment and the second distal segment is greater than an aggregate axial length of the second flexible structure and the second distal section.

11. The surgical instrument platform of claim 10, wherein a distal portion of the second distal segment consists of a third inner sub-tube and a fourth inner sub-tube, wherein the third inner sub-tube has an outer diameter that is not greater than an inner diameter of the second distal section, wherein the fourth inner sub-tube has an outer diameter that is greater than the inner diameter of the second distal section, and wherein an aggregate axial length of the third inner sub-tube and the second intermediate segment is greater than the aggregate axial length of the second distal section and the second flexible structure.

12. The surgical instrument platform of claim 3, wherein the wrist structure has two rotational degrees of freedom respectively about a fifth axis and a sixth axis, and
wherein the third flexible structure has two rotational degrees of freedom respectively about a seventh axis and an eighth axis,
wherein: the fifth axis is parallel to the seventh axis; the sixth axis is parallel to the eighth axis; and the fifth axis is perpendicular to the sixth axis,
wherein when the wrist structure is being driven by the manipulator structure to rotate about the fifth axis, the sensor member is configured to detect the rotation of the wrist structure about the fifth axis and to send a third detected rotation information to the control member, wherein the control member is configured to control the drive member to output a power based on the third detected rotation information, and wherein the drive member is configured to drive, through the transmission member, the third flexible structure to rotate about the seventh axis in a same direction as the rotation of the wrist structure, and
wherein when the wrist structure is being driven by the manipulator structure to rotate about the sixth axis, the sensor member is configured to detect the rotation of the wrist structure about the sixth axis and to send a fourth detected rotation information to the control member, wherein the control member is configured to control the drive member to output a power based on the fourth detected rotation information, and wherein the drive member is configured to drive, through the transmission member, the third flexible structure to rotate about the eighth axis in a same direction as the rotation of the wrist structure.

13. The surgical instrument platform of claim 3, wherein the manipulator structure has a roll degree of freedom about a ninth axis, wherein the end effector has a roll degree of freedom about a tenth axis, wherein the manipulator structure is rotatably disposed on the wrist structure and wherein the end effector is coupled to the third flexible structure,
wherein when the manipulator structure is rolling about the ninth axis, the sensor member is configured to detect the rolling of the manipulator structure and to send a detected rolling information of the manipulator structure to the control member, wherein the control member is configured to control the drive member to output a power based on the detected rolling information of the manipulator structure, and wherein the drive member is configured to drive, through the transmission member, the end effector to roll in a same direction as the rolling of the manipulator structure.

14. The surgical instrument platform of claim 2 or 3, wherein: the end effector comprises at least one tool arm; the manipulation end further comprises an opening and closing control structure disposed on the manipulator structure and being able to make an opening and closing movement,
wherein when the opening and closing control structure is making the opening and closing movement, the sensor member is configured to detect the opening and closing movement of the opening and closing control structure and to send the detected opening and closing movement information to the control member, wherein the control member is configured to control the drive member to output a power based on the detected opening and closing movement information, wherein the drive member is configured to drive, through the transmission member, the tool arm of the end effector to make an opening and closing movement, which occurs in a same manner as the opening and closing movement of the opening and closing control structure.

15. The surgical instrument platform of claim 3, wherein the sensor member comprises a number of sensors for detecting a movement of each of the manipulation end and the first flexible structure.

16. The surgical instrument platform of claim 15, wherein the sensor member comprises a first sensor, a second sensor, a third sensor, a fourth sensor, a fifth sensor, a sixth sensor and a seventh sensor,
wherein: the first sensor is configured to detect a pitch movement of the wrist structure; the second sensor is configured to detect a yaw movement of the wrist structure; the third sensor is configured to detect the opening and closing movement of the manipulation end; the fourth sensor is configured to detect a roll movement of the manipulator structure; the fifth sensor is configured to detect a pitch movement of the first flexible structure; the sixth sensor is configured to detect a yaw movement of the first flexible structure; and the seventh sensor is configured to detect a translational movement of the manipulation end.

17. The surgical instrument platform of claim 3, wherein: the transmission member comprises a first transmission part and a second transmission part; the first transmission part is configured to convert a movement of the first connecting structure into a movement detectable by the sensor member; and the second transmission part is coupled to the drive member to drive the second connecting structure to move.

18. The surgical instrument platform of claim 17, wherein the first transmission part comprises a first cable transmission assembly and a second cable transmission assembly,
wherein the first cable transmission assembly comprises a first transmission cable and a first cable pulley, wherein the first cable transmission assembly is configured to use the first transmission cable to convert a pitch movement of the first flexible structure about a first axis into a rotational movement of the first cable pulley, wherein the second cable transmission assembly comprises a second transmission cable and a second cable pulley, wherein the second cable transmission assembly is configured to use the second transmission cable to convert a yaw movement of the first flexible structure about a second axis into a rotational movement of the second cable pulley,
wherein: a proximal end of the first transmission cable is coupled to the first flexible structure; a distal end of the first transmission cable is wound through the first cable pulley and is then coupled to the first flexible structure; a proximal end of the second transmission cable is coupled to the first flexible structure; and a distal end of the second transmission cable is wound through the second cable pulley and is then coupled to the first flexible structure,
wherein the sensor member is configured to detect the rotational movement of the first cable pulley and the rotational movement of the second cable pulley and to send a detected rotation information of the first cable pulley and a detected rotation information of the second cable pulley to the control member, wherein the control member is configured to determine a rotational condition of the first flexible structure about a first axis based on the detected rotation information of the first cable pulley and to determine a rotational condition of the first flexible structure about a second axis based on the detected rotation information of the second cable pulley.

19. The surgical instrument platform of claim 17, wherein the first transmission part is further configured to convert a movement of the wrist structure into a movement detectable by the sensor member, and
wherein the first transmission part comprises a third cable transmission assembly and a fourth cable transmission assembly,
wherein the third cable transmission assembly comprises a third transmission cable and a third cable pulley, wherein the third cable transmission assembly is configured to use the third transmission cable to convert a pitch movement of the wrist structure about a fifth axis into a rotational movement of the third cable pulley, wherein a proximal end of the third transmission cable is coupled to the wrist structure, and wherein a distal end of the third transmission cable is wound through the third cable pulley and is then coupled to the wrist structure,
wherein the fourth cable transmission assembly comprises a fourth transmission cable and a fourth cable pulley, wherein the fourth cable transmission assembly is configured to use the fourth transmission cable to convert a yaw movement of the wrist structure about a sixth axis into a rotational movement of the fourth cable pulley, wherein a proximal end of the fourth transmission cable is coupled to the wrist structure, and wherein a distal end of the fourth transmission cable is wound through the fourth cable pulley and is then coupled to the wrist structure,
wherein the sensor member is configured to detect the rotational movement of the third cable pulley and the rotational movement of the fourth cable pulley, and to send a detected rotation information of the third cable pulley and a detected rotation information of the fourth cable pulley to the control member, wherein the control member is configured to determine a rotational condition of the wrist structure about the fifth axis based on the detected rotation information of the third cable pulley and to determine a rotational condition of the wrist structure about the sixth axis based on the detected rotation information of the fourth cable pulley.

20. The surgical instrument platform of claim 17, wherein the drive member comprises a first motor and a second motor, wherein the second transmission part comprises a fifth cable transmission assembly and a sixth cable transmission assembly, wherein the fifth cable transmission assembly is configured to drive a pitch movement of the second flexible structure, and wherein the sixth cable transmission assembly is configured to drive a yaw movement of the second flexible structure,
wherein the fifth cable transmission assembly comprises at least two fifth transmission cables, wherein a proximal end of each of the fifth transmission cables is coupled to the first motor and a distal end of each of the fifth transmission cables is coupled to the second flexible structure,
wherein the sixth cable transmission assembly comprises at least two sixth transmission cables, wherein a proximal end of each of the sixth transmission cables is coupled to the second motor and a distal end of each of the sixth transmission cables is coupled to the second flexible structures.

21. The surgical instrument platform of claim 17, wherein: the drive member comprises a third motor and a fourth motor; the second transmission part comprises a seventh cable transmission assembly and an eighth cable transmission assembly; the seventh cable transmission assembly is configured to drive a pitch movement of the third flexible structure; and the eighth cable transmission assembly is configured to drive a yaw movement of the third flexible structure,
wherein the seventh cable transmission assembly comprises at least two seventh transmission cables, wherein a proximal end of each of the seventh transmission cables is coupled to the third motor, and wherein a distal end of each of the seventh transmission cables passes through the second flexible structure and is then coupled to the third flexible structure,
wherein the eighth cable transmission assembly comprises at least two eighth transmission cables, wherein a proximal end of each of the eighth transmission cables is coupled to the fourth motor, and wherein a distal end of each of the eighth transmission cables passes through the second flexible structure and is then coupled to the third flexible structure.

22. The surgical instrument platform of claim 17, wherein the first transmission part comprises a first rack and pinion transmission mechanism comprising a first rack and a first pinion in meshing engagement with the first rack, wherein the first pinion is coupled to the drive member, wherein the first rack is coupled to the first connecting structure, and wherein the first rack and pinion transmission mechanism is configured to convert a translation of the first connecting structure into a rotational movement of the first pinion,
wherein the sensor member is configured to detect the rotational movement of the first pinion and to send a detected rotation information of the first pinion to the control member, wherein the control member is configured to control the drive member to output a power based on the detected rotation information of the first pinion, and wherein the drive member is configured to drive the second connecting structure to move through the second transmission part.

23. The surgical instrument platform of claim 17, wherein the first transmission part is further configured to convert a roll movement of the manipulator structure into a movement detectable by the sensor member, and
wherein the first transmission part comprises a first shaft assembly comprising a first flexible shaft and a first rotary wheel, wherein a proximal end of the first flexible shaft is coupled to the manipulator structure, a distal end of the first flexible shaft coupled to the first rotary wheel,
wherein the sensor member is configured to detect a rotation of the first rotary wheel and to send a detected rotation information of the first rotary wheel to the control member, wherein the control member is configured to control the drive member to output a power based on the detected rotation information of the first rotary wheel, and wherein the drive member is configured to drive the end effector to roll through the second transmission part.

24. The surgical instrument platform of claim 17, wherein the second transmission part comprises a second rack and pinion transmission mechanism comprising a second rack and a second pinion in meshing engagement with the second rack, wherein the second pinion is coupled to the drive member, and wherein the second rack is coupled to the second connecting structure,
wherein the sensor member is configured to detect a rotation of the second pinion and to send a detected rotation information of the second pinion to the control member, wherein the control member is configured to control the drive member to output a power based on the detected rotation information of the second pinion, and wherein the drive member is configured to drive the second connecting structure to move through the second rack.

25. The surgical instrument platform of claim 17, wherein the second transmission part comprises a second shaft assembly comprising a second flexible shaft, wherein a proximal end of the second flexible shaft is coupled to the drive member and a distal end of the second flexible shaft is coupled to the end effector,
wherein the sensor member is configured to detect a roll movement of the manipulator structure and to send a detected roll information of the manipulator structure to the control member, wherein the control member is configured to control the drive member to output a power based on the detected roll information of the manipulator structure, and wherein the drive member is configured to transmit the roll movement to the end effector through the second flexible shaft, thereby driving the end effector to roll.

26. The surgical instrument platform of claim 17, wherein the second transmission part comprises a first adapter, a flexible transmission mechanism for opening and closing movement and a second adapter that are sequentially connected,
wherein the first adapter is coupled to the drive member and is configured to convert a rotational movement of the drive member into an axial movement of the flexible transmission mechanism, wherein the second adapter is coupled to a tool arm of the end effector and is configured to convert the axial movement of the flexible transmission mechanism into an opening and closing movement of the tool arm, and wherein the flexible transmission mechanism, the first adapter and the second adapter are configured to cause the tool arm of the end effector to move in a same manner as the opening and closing control structure,
wherein the sensor member is configured to detect an opening and closing movement of the opening and closing control structure and to send a detected opening and closing information of the opening and closing control structure to the control member, wherein the control member is configured to control the drive member to output a power based on the detected opening and closing information of the opening and closing control structure, and wherein the drive member is configured to drive the opening and closing movement of the end effector through the first adapter, the flexible transmission mechanism and the second adapter.

27. The surgical instrument platform of claim 18, wherein the second cable transmission assembly further comprises an elastic element for compensating for a curvature of the second cable transmission assembly and thereby maintaining the surgical instrument in a deployed configuration.

28. The surgical instrument platform of claim 1, wherein the instrument platform comprises a housing, on which an interface coupled to the end of the mechanical arm is formed.

29. The surgical instrument platform of claim 1, wherein the mechanical arm comprises at least six joints to provide at least six degrees of freedom, wherein the mechanical arm includes at least one translational joint and at least five rotational joints, wherein two rotational joints and one translational joint work together to adjust the position of the instrument assembly, and wherein three other rotational joints are configured to adjust the orientation of the instrument assembly.

30. The surgical instrument platform of claim 29, wherein the mechanical arm comprises seven joints coupled sequentially from a proximal end to a distal end of the mechanical arm, which are a first translational joint, a first rotational joint, a second rotational joint, a third rotational joint, a second translational joint, a fourth rotational joint and a fifth rotational j oint,
wherein: the first translational joint has an axis of translation that is collinear with an axis of rotation of the first rotational joint; the axis of rotation of the first rotational joint is parallel to an axis of rotation of the second rotational joint; the third rotational joint has an axis of rotation that is collinear with an axis of translation of the second translational joint; and the fourth rotational joint has an axis of rotation that is perpendicular to an axis of rotation of the fifth rotational joint.

31. The surgical instrument platform of claim 30, wherein the axes of rotation of the third, fourth and fifth rotational joints intersect at a single point.

32. The surgical instrument platform of claim 1, wherein the instrument platform comprises a housing, on which the surgical instrument is detachably arranged, wherein a sleeve is provided outside of a distal end of the housing, wherein the sleeve extends outwardly from the distal end of the housing, the sleeve configured to be deployed at an incision made in a patient.

33. The surgical instrument platform of claim 32, wherein the housing is provided therein with a number of instrument channels extending from a proximal end of the housing to a distal end of the sleeve, wherein a proximal portion of the surgical instrument extends into the housing from the proximal end thereof and is accommodated in the corresponding instrument channel in the housing, and wherein a distal portion of the surgical instrument is accommodated in the instrument channel from the distal end thereof and extends through and protrudes out of the sleeve.

34. The surgical instrument platform of claim 33, wherein the instrument platform further comprises a tool axis, a plane of symmetry and a working plane, wherein the working plane is perpendicular to the plane of symmetry, wherein the working plane crosses axes of at least two of the instrument channels, wherein the instrument channels are arranged in symmetry with respect to the plane of symmetry, and wherein the tool axis is defined as an intersection of the plane of symmetry and the working plane,
wherein in an initial configuration, the distal portion of the surgical instrument is linear in shape and is parallel to the tool axis, and wherein the proximal portion of the surgical instrument is curved in shape, and
wherein in a deployed configuration, the distal portion of the surgical instrument is C-shaped and is oriented in the working plane, and wherein the proximal portion of the surgical instrument is more curved than in the initial configuration.

35. A surgical instrument, comprising a manipulation end, a connection assembly and an end effector, which are connected sequentially from a proximal end to a distal end of the surgical instrument, wherein: the connection assembly comprises a proximal first connecting structure and a distal second connecting structure; a proximal end of the first connecting structure is coupled to the manipulation end, and a distal end of the first connecting structure is coupled to an instrument platform of a surgical instrument platform; a distal end of the second connecting structure is coupled to the end effector, and a proximal end of the second connecting structure is coupled to the instrument platform,
wherein the surgical instrument further comprises a sensor member, a drive member, a transmission member and a control member, wherein: the sensor member is configured to detect a movement of the first connecting structure and to send a detected movement information of the first connecting structure to the control member; the control member is configured to control the drive member to output a power based on the detected movement information of the first connecting structure; and the drive member is configured to drive a movement of the second connecting structure through the transmission member.
